# EUROPEAN PATENT SPECIFICATION

(11) **EP 3 228 303 B1**
(45) Date of publication and mention of the grant of the patent: **18.03.2020**
(21) Application number: 15866022.5
(22) Date of filing: 17.11.2015
(51) Int. Cl.: A61K 8/365, A61K 8/49, A61Q 5/04, A61Q 5/06

(54) **AGENT FOR HAIR DEFORMING TREATMENT**
BEHANDLUNGSMITTEL ZUR HAARVERFORMUNG
AGENT DE TRAITEMENT DE DÉFORMATION CAPILLAIRE

(30) Priority: 05.12.2014 JP 2014247484
(43) Date of publication of application: 11.10.2017
(73) Proprietor: Kao Corporation, Chuo-ku Tokyo 103-8210 (JP)
(72) Inventor: FURUKAWA, Junichi, Tokyo 131-8501 (JP); TOKUNAGA, Shinichi, Tokyo 131-8501 (JP); TSUCHIYA, Masaru, Haga-gun Tochigi 321-3497 (JP)
(74) Representative: Hoffmann Eitle
(86) International application number: PCT/JP2015/082283
(87) International publication number: WO 2016/088550

(56) References cited:
- WO-A2-2014/068101
- WO-A2-2014/068101
- DE-A1- 3 320 539
- DE-A1-102011 017 519
- DE-C1- 19 735 865
- DE-C1- 19 810 120
- JP-A- 2005 194 261
- JP-A- 2013 520 468
- JP-A- 2013 531 046
- BOGA, C. ET AL.: 'Formaldehyde replacement with glyoxylic acid in semipermanent hair straightening: a new and multidisciplinary investigation' INTERNATIONAL JOURNAL OF COSMETIC SCIENCE vol. 36, 11 August 2014, pages 459 - 470, XP055173854

## Description

### [Field of the Invention]

The present invention relates to an agent for hair deforming treatment capable of semi-permanently or permanently deforming the shape of hair.

### [Background of the Invention]

As methods for semi-permanently or permanently deforming the shape of hair, there have been a method of using a reducing agent, as in the case of the formation of a permanent wave, and a method of using an agent for a strongly-alkaline treatment agent having a pH 12 to 14, including an alkali relaxer as a representative example. However, it has been well known that these methods impart a severe impact on hair, and damage the hair. In addition, in recent years, as a method for semi-permanently or permanently deforming the hair with less damage to the hair, a hair-relaxing method using a large amount of formaldehyde has been developed. However, such a method of using highly toxic formaldehyde must be handled carefully, because of its high volatility, and thus, the method is not always a preferred method of hair treatment.

Hence, a hair-relaxing method, which does not impart damage to hair and does not use formaldehyde, and which is safer to a human body, has been desired. For example, Patent Document 1 discloses a technology for applying α-keto acid, and particularly, glyoxylic acid to hair, and then subjecting the hair to a heat treatment with a flat iron at 200°C ± 50°C, to convert very curly hair to straight hair. Moreover, Patent Document 2 discloses a method of converting hair to straight hair, using a crosslinking agent, and particularly, dialdehyde.

On the other hand, the use of flavonoids including catechin as a typical example has been studied in the field of hair cosmetics. For example, Patent Document 2 describes that the conditioning performance of a hair cosmetic is improved, when epigallocatechin, as well as a crosslinking agent, is mixed into the hair cosmetic. In addition, Patent Document 3 describes that a hair cosmetic comprising flavonoid, hydrogen peroxide and enzyme repairs and reinforces hair. Moreover, Patent Document 4 describes that carbonyl such as catechin or glyoxal is used to improve hair dyeability and to suppress unpleasant odor in a hair dye comprising dihydroxyacetone.
[Patent Document 1] EP 2538916 A
[Patent Document 2] WO 2009/035970
[Patent Document 3] WO 2013/087644

DE 198 10 120 and DE 197 35 865 relate to hair cosmetic compositions such as leave-on conditioners comprising a green tea extract, which may additionally include glyoxylic acid as a buffering agent.

DE 33 20 539 describes flavonoid-containing hair cosmetic compositions.

WO 2014/068101 is directed to a method for bleaching and dyeing the hair, involving a step of bleaching the hair with a composition comprising an oxidizing agent and an alkaline agent, which is directly followed by a step of straightening the hair using a carboxylic acid compound of a specific formula such as glyoxylic acid.

### Summary of the Invention

The present invention provides an agent for hair deforming treatment comprising the following components (A), (B) and (C):
(A) glyoxylic acid, or a hydrate or a salt thereof,
(B) one or two or more member selected from the compounds represented by the following formula (1): 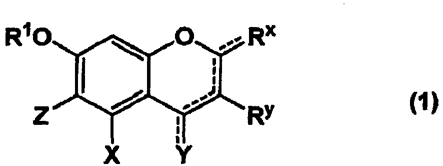 wherein
   R¹ represents a hydrogen atom or a methyl group,
   X represents a hydrogen atom, a hydroxy group, or a methoxy group,
   Y represents a hydrogen atom, an oxygen atom, a hydroxy group, or a methoxy group,
   Z represents a hydrogen atom, or a straight-chain or branched-chain alkyl group or alkenyl group containing from 1 to 5 carbon atoms,
   R^{x} represents a hydrogen atom, an oxygen atom, a hydroxy group, a methoxy group, or an aromatic hydrocarbon group, which is optionally substituted with up to three hydroxy groups or methoxy groups and which optionally forms a condensed ring with 1,3-dioxolane,
   R^{y} represents a hydrogen atom, a hydroxy group, a methoxy group, or an aromatic hydrocarbon group, which is optionally substituted with up to three hydroxy groups or methoxy groups and which optionally forms a condensed ring with 1,3-dioxolane, or an arylcarbonyloxy group or aralkylcarbonyloxy group, which is optionally substituted with up to three hydroxy groups or methoxy groups, and
   the dashed line optionally represents a double bond,
   provided that the dashed line and the solid line, which are adjacent to R^{x} or Y, each represent a double bond only when R^{x} or Y is an oxygen atom, and otherwise these lines each represent a single bond, and provided that,
   Z represents a straight-chain or branched-chain alkyl group or alkenyl group containing from 1 to 5 carbon atoms, only when R^{x} or R^{y} is an o,p-dihydroxy aromatic hydrocarbon group, and otherwise Z is a hydrogen atom, and
(C) water,
wherein the molar ratio of the content of the component (B) to the content of the component (A), (B)/(A), is 0.01 or more and 5 or less; and
wherein the content of the component (A) is 2.5 mass% or more and 30 mass% or less.

Moreover, the present invention provides a method of hair treatment for semi-permanently or permanently deforming the shape of hair, which comprises the following steps (i) and (ii):
(i) applying the above described agent for hair deforming treatment to hair, and then allowing the agent to penetrate into the hair, and
(ii) heating and shaping the hair into which the agent for hair deforming treatment has penetrated.

### [Description of Embodiments]

In the method described in Patent Document 1 or 2, although it could convert the hair to straight hair semi-permanently, it could not provide a semi-permanently or permanently wavy or curly shape to the hair. In addition, when the once formed semi-permanent or permanent straight shape intends to be converted to another semi-permanent or permanent shape such as a wavy or curly shape, it is necessary to perform again the conventional operation of using a reducing agent. Thus, excessive amounts of time and efforts are required, and further, hair may be damaged.

Moreover, in the technology described in Patent Document 2, since the active ingredient has genotoxicity, even if the effect of deforming the shape of hair intends to be increased, it has been difficult to use the active ingredient in a large amount. Furthermore, there has also been a case where a hair cosmetic must be maintained for 48 hours without being washed away from hair, after it has been applied to the hair. Thus, enormous amounts of time and efforts have been required, and it has not been convenient to use this hair cosmetic. Further, even if flavonoid has simply been applied to hair, as described in Patent Documents 3 and 4, semi-permanent deformation of the shape of hair has not been realized.

Accordingly, the present invention relates to an agent for hair deforming treatment and a method of hair treatment, which are safe for human bodies and have less damage to hair when the agent is applied to hair, are able to semi-permanently or permanently provide not only the shape of straight hair, but also a wavy or curly shape, and further, are able to easily convert the once formed hair shape to another semi-permanent or permanent hair shape, without using agent for hair deforming treatments such as a reducing agent, and without damaging to the hair.

The present inventors have found that a hair treatment agent comprising glyoxylic acid and a specific catechin analog cannot only semi-permanently provide a straight hair shape or a curly or wavy hair shape to hair, but also deform, semi-permanently or permanently, hair that has once been treated with this hair treatment agent, to any completely different, any hair shape, by only using a heating means such as a hair iron or a curler, without treating the hair with a hair treatment agent such as a reducing agent, thereby completing the present invention.

The agent for hair deforming treatment of the present invention is highly safe for human bodies, has less damage to hair, and can deform the shape of hair semi-permanently or permanently, and the hair deformed with the present agent for hair deforming treatment does not lose its effect even if the hair is washed with, for example, a shampoo. In addition, once the present agent for hair deforming treatment is applied to hair, it is not necessary to allow a hair treatment agent to penetrate into the hair again, and the hair can be freely and repeatedly deformed semi-permanently or permanently only by heating to the hair. Moreover, the hair, which has been arbitrarily repeatedly deformed by being heated, still has a high hair washing resistance of the shape of the hair, and thus the shape of the hair does not lose its effect by shampoo, water, etc.

In the present invention, "semi-permanent or permanent hair deformation" means that hair has extremely excellent hair washing resistance, and that the shape of the hair is maintained, even if shampooing is repeatedly carried out thereon. Specifically, it means that, when the deformed hair is washed with shampoo, and the shampoo is then fully washed away with water, and the hair is then naturally dried, the shape of hair is maintained before and after shampooing. It is to be noted that the expression "the shape of hair is maintained" means that, for example, in the case of wavy hair, the number of waves is not substantially different before and after shampooing, and in the case of straight hair, wavy or curly hair is not substantially generated as a result of shampooing.

In the present invention, "hair deformation" means deformation of hair, which is not caused by the cleavage and recombination of the S-S bond of proteins in hair, and it includes deformation of straight hair into, for example, curly hair, and also, deformation of hair which has been deformed into wavy or curly hair, or naturally curly hair, to straight hair.

### [Component (A): glyoxylic acid, or a hydrate or salt thereof]

The component (A) includes not only glyoxylic acid, but also a hydrate of the glyoxylic acid and a salt of the glyoxylic acid. An example of the hydrate of the glyoxylic acid is a glyoxylic acid monohydrate. Examples of the salt of the glyoxylic acid include an alkaline metal salt of the glyoxylic acid and an alkaline-earth metal salt of the glyoxylic acid. Examples of the alkaline metal salt include a lithium salt, a sodium salt, and a potassium salt. Examples of the alkaline-earth metal salt include a magnesium salt and a calcium salt.

From the viewpoint of achieving a more significant change in the shape of hair after the treatment of hair with the agent for hair deforming treatment of the present invention, achieving more excellent hair washing resistance of the shape of hair, achieving a more significant change in the shape of hair upon semi-permanent re-deformation of the shape of hair by heating, and also achieving more excellent hair washing resistance of the shape of hair after completion of the re-deformation, the content of the component (A) in the agent for hair deforming treatment of the present invention is, relative to the content of glyoxylic acid, 2.5 mass% or more, more preferably 3 mass% or more, even more preferably 4 mass% or more, and further preferably 5 mass% or more, and in addition to the aforementioned viewpoint, also from the viewpoint of suppression of irritation to the skin, the content of the component (A) is, relative to the content of glyoxylic acid, 30 mass% or less, more preferably 25 mass% or less, even more preferably 20 mass% or less, even more preferably 15 mass% or less, and even more preferably 12 mass% or less.

### [Component (B): compound represented by formula (1)]

The component (B) is a compound represented by the following formula (1): 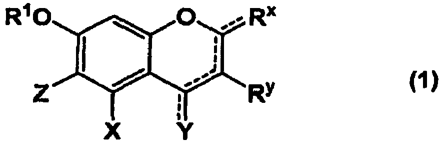 wherein
R¹ represents a hydrogen atom or a methyl group,
X represents a hydrogen atom, a hydroxy group, or a methoxy group,
Y represents a hydrogen atom, an oxygen atom, a hydroxy group, or a methoxy group,
Z represents a hydrogen atom, or a straight-chain or branched-chain alkyl group or alkenyl group containing from 1 to 5 carbon atoms,
R^{x} represents a hydrogen atom, an oxygen atom, a hydroxy group, a methoxy group, or an aromatic hydrocarbon group, which is optionally substituted with up to three hydroxy groups or methoxy groups and which optionally forms a condensed ring with 1,3-dioxolane,
R^{y} represents a hydrogen atom, a hydroxy group, a methoxy group, or an aromatic hydrocarbon group, which is optionally substituted with up to three hydroxy groups or methoxy groups and which optionally forms a condensed ring with 1,3-dioxolane, or an arylcarbonyloxy group or aralkylcarbonyloxy group, which is optionally substituted with up to three hydroxy groups or methoxy groups, and
the dashed line optionally represents a double bond,
provided that the dashed line and the solid line, which are adjacent to R^{x} or Y, each represent a double bond only when R^{x} or Y is an oxygen atom, and otherwise these lines each represent a single bond, and provided that,
Z represents a straight-chain or branched-chain alkyl group or alkenyl group containing from 1 to 5 carbon atoms, only when R^{x} or R^{y} is an o,p-dihydroxy aromatic hydrocarbon group, and otherwise Z is a hydrogen atom.

Examples of the straight-chain or branched-chain alkyl group or alkenyl group containing from 1 to 5 carbon atoms, which is represented by Z, include a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, a neopentyl group, a 1-methylpentyl group, a vinyl group, an allyl group, and a butenyl group.

Examples of the aromatic hydrocarbon group, which is represented by R^{x} or R^{y}, include a phenyl group and a naphthyl group. An example of the aromatic hydrocarbon group, which forms a condensed ring with 1,3-dioxolane is a 1,3-benzodioxol-5-yl group.

An example of the arylcarbonyloxy group represented by R^{y} is a benzoyloxy group, and examples of the aralkylcarbonyloxy group represented by R^{y} include a benzylcarbonyloxy group, a phenylethylcarbonyloxy group, a phenylpropylcarbonyloxy group, and a phenylbutylcarbonyloxy group.

The compound represented by the formula (1) specifically includes the following (1-1) to (1-5):

### (1-1) Flavanol compounds represented by the following formula (1-1) :

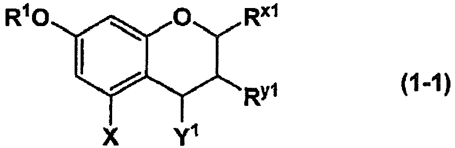

wherein
R¹ and X are defined as described above,
Y¹ represents a hydrogen atom, a hydroxy group, or a methoxy group,
R^{x1} represents an aromatic hydrocarbon group, which is optionally substituted with up to three hydroxy groups or methoxy groups and which optionally forms a condensed ring with 1,3-dioxolane,
R^{y1} represents a hydrogen atom, a hydroxy group, a methoxy group, or an aromatic hydrocarbon group, which is optionally substituted with up to three hydroxy groups or methoxy groups and which optionally forms a condensed ring with 1,3-dioxolane, or an arylcarbonyloxy group or aralkylcarbonyloxy group, which is optionally substituted with up to three hydroxy groups or methoxy groups.

### (1-2) Flavanone compounds or flavanonol compounds represented by the following formula (1-2):

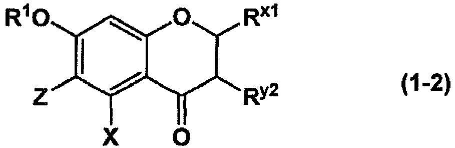

wherein R¹, X, Z and R^{x1} are defined as described above, and R^{y2} represents a hydrogen atom, a hydroxy group, or a methoxy group,
provided that Z represents a straight-chain or branched-chain alkyl group or alkenyl group containing from 1 to 5 carbon atoms, only when R^{x1} is an o,p-dihydroxy aromatic hydrocarbon group, and otherwise Z is a hydrogen atom.

### (1-3) Flavone compounds or flavonol compounds represented by the following formula (1-3):

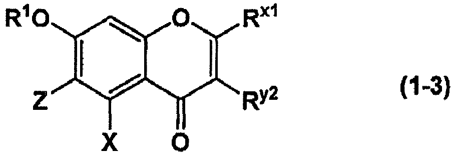

wherein R¹, X, Z, R^{x1} and R^{y2} are as defined above,
provided that Z represents a straight-chain or branched-chain alkyl group or alkenyl group containing from 1 to 5 carbon atoms, only when R^{x1} is an o,p-dihydroxy aromatic hydrocarbon group, and otherwise z is a hydrogen atom.

### (1-4) Isoflavone compounds or isoflavane compounds represented by the following formula (1-4):

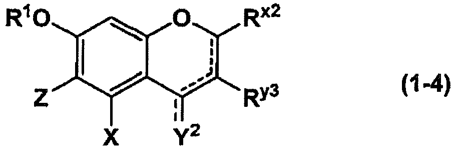

wherein
R¹, X, Z and the dashed line are as defined above,
Y² represents a hydrogen atom or an oxygen atom,
R^{x2} represents a hydrogen atom, a hydroxy group, or a methoxy group, and
R^{y3} represents an aromatic hydrocarbon group, which is optionally substituted with up to three hydroxy groups or methoxy groups and which optionally forms a condensed ring with 1,3-dioxolane,
provided that the dashed line and the solid line, which are adjacent to Y², each represent a double bond only when Y² is an oxygen atom, and otherwise these lines each represent a single bond, and provided that,
Z represents a straight-chain or branched-chain alkyl group or alkenyl group containing from 1 to 5 carbon atoms, only when R^{y3} is an o,p-dihydroxy aromatic hydrocarbon group, and otherwise Z is a hydrogen atom.

### (1-5) Coumarin compounds represented by the following formula (1-5) :

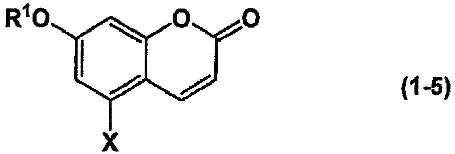

wherein R¹ and X are as defined above.

As the compounds represented by the formula (1-1), the following (1-1-A) to (1-1-C) are preferable.

### (1-1-A) Flavan-3-ol compounds represented by the following formula (1-1-A) :

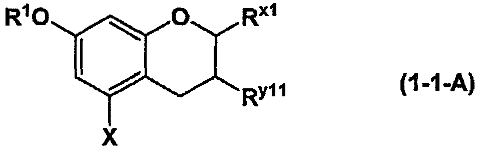

wherein R¹, X and R^{x1} are as defined above, and R^{y11} represents a hydroxy group, a methoxy group, or an aromatic hydrocarbon group, which is optionally substituted with up to three hydroxy groups or methoxy groups and which optionally forms a condensed ring with 1,3-dioxolane, or an arylcarbonyloxy group or aralkylcarbonyloxy group, which is optionally substituted with up to three hydroxy groups or methoxy groups.

As flavan-3-ol compounds represented by the formula (1-1-A), a compound, wherein, in the above formula, R¹ and X are as defined above, R^{x1} represents an aromatic hydrocarbon group, which is optionally substituted with up to three hydroxy groups or methoxy groups, and R^{y} represents a hydrogen atom, a hydroxy group, a methoxy group, or an arylcarbonyloxy group or aralkylcarbonyloxy group, which is optionally substituted with up to three hydroxy groups or methoxy groups, is preferable.

Examples of the compounds corresponding to the (1-1-A) include catechin, epicatechin, epigallocatechin, meciadanol, afzelechin, epiafzelechin, catechin gallate, epicatechin gallate, epigallocatechin gallate, phylloflavan, fisetinidol, guibourtinidol, and robinetinidol.

### (1-1-B) Flavan-4-ol compounds represented by the following formula (1-1-B) :

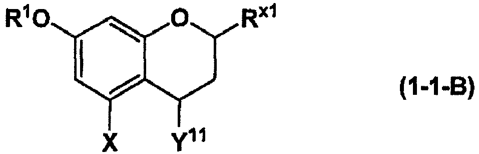

wherein R¹, X and R^{x1} are as defined above, and Y¹¹ represents a hydroxy group or a methoxy group.

As flavan-4-ol compounds represented by the formula (1-1-B), a compound, wherein, in the above formula, R¹ and X are as defined above, Y¹¹ represents a hydroxy group, and R^{x1} represents an aromatic hydrocarbon group, which is optionally substituted with up to three hydroxy groups or methoxy groups, is preferable.

Examples of the compounds corresponding to the (1-1-B) include apiforol and luteoforol.

### (1-1-C) Flavan-3,4-diol compounds represented by the following formula (1-1-C):

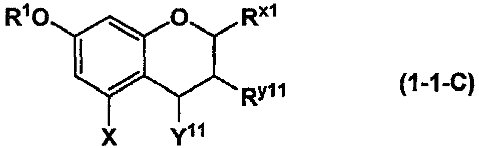

wherein R¹, X, Y¹¹, R^{x1} and R^{y11} are as defined above.

As the flavan-3,4-diol compounds represented by the formula (1-1-C), a compound, wherein, in the above formula, R¹ and X are as defined above, Y¹¹ represents a hydroxy group or a methoxy group, R^{x1} represents an aromatic hydrocarbon group, which is optionally substituted with up to three hydroxy groups or methoxy groups, and R^{y1} represents a hydroxy group or a methoxy group, is preferable.

Examples of the compounds corresponding to the (1-1-C) include leucocyanidin, leucodelphinidin, leucopelargonidin, leucopeonidin, and leucofisetinidin.

As the compounds represented by the formula (1-2), the following (1-2-A) and (1-2-B) are preferable.

### (1-2-A) Flavanone compounds represented by the following formula (1-2-A) :

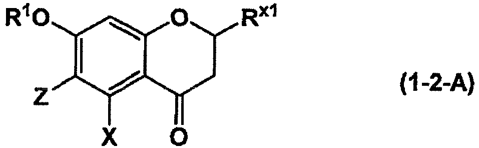

wherein R¹, X, Z and R^{x1} are as defined above,
provided that Z represents a straight-chain or branched-chain alkyl group or alkenyl group containing from 1 to 5 carbon atoms, only when R^{x1} represents an o,p-dihydroxy aromatic hydrocarbon group, and otherwise Z is a hydrogen atom.

As the flavanone compounds represented by the formula (1-2-A), a compound, wherein, in the above formula, R¹ and X are as defined above, Z represents a hydrogen atom, and R^{x1} represents an aromatic hydrocarbon group, which is optionally substituted with up to three hydroxy groups or methoxy groups, is preferable.

Examples of the compounds corresponding to the (1-2-A) include eriodictyol, naringenin, pinocembrin, hesperetin, homoeriodictyol, isosakuranetin, sterubin, sakuranetin, alpinetin, and butin.

### (1-2-B) Flavanonol compounds represented by the following formula (1-2-B) :

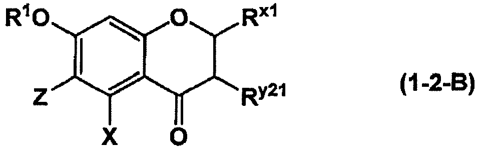

wherein R¹, X, Z and R^{x1} are as defined above, and R^{y21} represents a hydroxy group or a methoxy group,
provided that Z represents a straight-chain or branched-chain alkyl group or alkenyl group containing from 1 to 5 carbon atoms, only when R^{x1} is an o,p-dihydroxy aromatic hydrocarbon group, and otherwise Z is a hydrogen atom.

As the flavanonol compounds represented by the formula (1-2-B), a compound, wherein, in the above formula, R¹ and X are as defined above, Z represents a hydrogen atom, R^{x1} represents an aromatic hydrocarbon group, which is optionally substituted with up to three hydroxy groups or methoxy groups, and R^{y1} represents a hydroxy group or a methoxy group, is preferable.

Examples of the compounds corresponding to the (1-2-B) include aromadendrin, taxifolin, and dihydrokaempferide.

As the compounds represented by the formula (1-3), the following (1-3-A) and (1-3-B) are preferable.

### (1-3-A) Flavone compounds represented by the following formula (1-3-A):

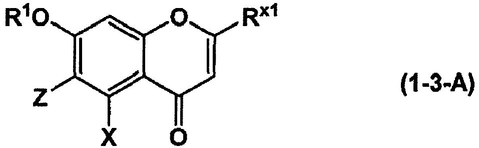

wherein R¹, X, Z and R^{x1} are as defined above,
provided that Z represents a straight-chain or branched-chain alkyl group or alkenyl group containing from 1 to 5 carbon atoms, only when R^{x1} is an o,p-dihydroxy aromatic hydrocarbon group, and otherwise Z is a hydrogen atom.

As the flavone compounds represented by the formula (1-3-A), a compound, wherein, in the above formula, R¹ and X are as defined above, Z represents a hydrogen atom, and R^{x1} represents an aromatic hydrocarbon group, which is optionally substituted with up to three hydroxy groups or methoxy groups, is preferable.

Examples of the compounds corresponding to the (1-3-A) include luteolin, apigenin, chrysin, norartocarpetin, tricetin, diosmetin, acacetin, chrysoeriol, genkwanin, techtochrysin, tricin, 4',7-dihydroxyflavone, and pratol.

### (1-3-B) Flavonol compounds represented by the following formula (1-3-B) :

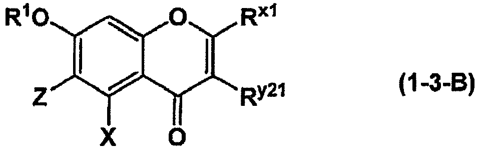

wherein R¹, X, Z, R^{x1} and R^{y21} are as defined above,
provided that Z represents a straight-chain or branched-chain alkyl group or alkenyl group containing from 1 to 5 carbon atoms, only when R^{x1} is an o,p-dihydroxy aromatic hydrocarbon group, and otherwise Z is a hydrogen atom.

As the flavonol compounds represented by the formula (1-3-B), a compound, wherein, in the above formula, R¹ and X are as defined above, Z represents a hydrogen atom, R^{x1} represents an aromatic hydrocarbon group, which is optionally substituted with up to three hydroxy groups or methoxy groups, and R^{y1} represents a hydroxy group or a methoxy group, is preferable.

Examples of the compounds corresponding to the (1-3-B) include quercetin, myricetin, morin, kaempferol, galangin, kaempferide, tamarixetin, laricitrin, annulatin, isorhamnetin, syringetin, rhamnetin, europetin, azaleatin, 5-O-methylmyricetin, retusin, pachypodol, rhamnazin, ayanin, ombuin, and fisetin.

As the compounds represented by the formula (1-4), the following (1-4-A) to (1-4-C) are preferable.

### (1-4-A) Isoflavone compounds represented by the following formula (1-4-A) :

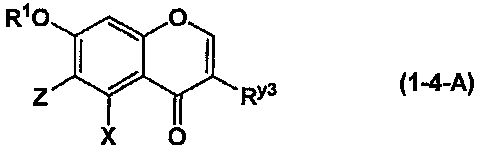

wherein R¹, X, Z and R^{y3} are as defined above,
provided that Z represents a straight-chain or branched-chain alkyl group or alkenyl group containing from 1 to 5 carbon atoms, only when R^{y3} is an o,p-dihydroxy aromatic hydrocarbon group, and otherwise Z is a hydrogen atom.

As the isoflavone compounds represented by the formula (1-4-A), a compound, wherein, in the above formula, R¹ and X are as defined above, Z represents a hydrogen atom, or a straight-chain or branched-chain alkyl group or alkenyl group containing from 1 to 5 carbon atoms, and R^{y3} represents an aromatic hydrocarbon group, which is optionally substituted with up to three hydroxy groups or methoxy groups and which optionally forms a condensed ring with 1,3-dioxolane, is preferable.

Examples of the compounds corresponding to the (1-4-A) include genistein, daidzein, orobol, biochanin A, pratensein, 5-O-methylgenistein, prunetin, calycosin,' formononetin, 7-O-methylluteone, luteone, and pseudobaptigenin).

### (1-4-B) Isoflavane compounds represented by the following formula (1-4-B) :

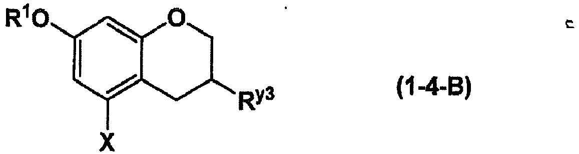

wherein R¹, X and R^{y3} are as defined above.

As the isoflavane compounds represented by the formula (1-4-B), a compound, wherein, in the above formula, R¹ and X are as defined above, and R^{y3} represents an aromatic hydrocarbon group, which is optionally substituted with up to three hydroxy groups or methoxy groups and which optionally forms a condensed ring with 1,3-dioxolane, is preferable.

An example of the compounds corresponding to the (1-4-B) is equol.

### (1-4-C) Isoflavene compounds represented by the following formula (1-4-C) :

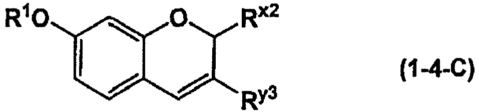

wherein R¹, R^{x2} and R^{y3} are as defined above.

As the isoflavene compounds represented by the formula (1-4-C), a compound, wherein, in the above formula, R¹ and R^{x2} are as defined above, and R^{y3} represents an aromatic hydrocarbon group, which is optionally substituted with up to three hydroxy groups or methoxy groups and which optionally forms a condensed ring with 1,3-dioxolane, is preferable.

Examples of the compounds corresponding to the (1-4-C) include haginin D, haginin E, and 2-methoxyjudaicin.

An example of the compounds corresponding to the (1-5) is umbelliferone.

Among these compounds, preferred examples of the compound represented by the formula (1) include flavan-3-ol compounds represented by the formula (1-1-A), flavonol compounds represented by the formula (1-3-B), flavanone compounds represented by the formula (1-2-A), flavone compounds represented by the formula (1-3-A), isoflavone compounds represented by the formula (1-4-A), isoflavane compounds represented by the formula (1-4-B), and coumarin compounds represented by the formula (1-5), More specifically, for example, catechin, epicatechin, epigallocatechin, catechin gallate, epicatechin gallate, epigallocatechin gallate, quercetin, morin, hesperetin, naringenin, chrysin, daidzein, equol, and umbelliferone are preferable. Among others, catechin, epigallocatechin, epigallocatechin gallate, naringenin, and equol are more preferable. Furthermore, a mixture comprising the above described compounds, such as a green tea extract, can also be used.

Moreover, the molecular weight of the compound represented by the formula (1) is preferably 150 or more. Furthermore, from the viewpoint of permeability into hair, it is preferably 1000 or less, more preferably 700 or less, and even more preferably 500 or less.

The component (B) can be used alone or in combination of two or more components. The use of two or more components is more preferable. From the viewpoint of achieving a more significant change in the shape of hair after the treatment of hair with the hair treatment agent of the present invention, achieving more excellent hair washing resistance of the shape of hair, achieving a more significant change in the shape of hair upon semi-permanent re-deformation of the shape of hair by heating, and also achieving more excellent hair washing resistance of the shape of hair after completion of the re-deformation, the content of the component (B) in the agent for hair deforming treatment of the present invention is preferably 0.2 mass% or more, more preferably 0.5 mass% or more, even more preferably 1 mass% or more, and further preferably 1.5 mass% or more, and also, from the viewpoint of formulation mixing properties, it is preferably 30 mass% or less, more preferably 25 mass% or less, even more preferably 23 mass% or less, and further preferably 20 mass% or less.

From the viewpoint of, because of a condensate of the component (A) and the component (B) formed in hair, achieving a more significant change in the shape of hair after the treatment of hair with the agent for hair deforming treatment of the present invention, achieving more excellent hair washing resistance of the shape of hair, achieving a more significant change in the shape of hair upon semi-permanent re-deformation of the shape of hair by heating, and also achieving more excellent hair washing resistance of the shape of hair after completion of the re-deformation, the molar ratio of the content of the component (B) to the content of the component (A) in the agent for hair deforming treatment of the present invention, (B)/(A), is 0.01 or more, preferably 0.1 or more, more preferably 0.3 or more, and even more preferably 0.5 or more, and also, it is 5 or less, preferably 4 or less, more preferably 3 or less, even more preferably 2 or less, and further preferably 1.5 or less.

### [Component (C) : water]

The agent for hair deforming treatment of the present invention uses water as a medium. Moreover, in addition to water, lower alcohol containing from 1 to 3 carbon atoms, such as methanol or ethanol, can be used in combination with water, as necessary. In this case, the content of the lower alcohol containing from 1 to 3 carbon atoms in the agent for hair deforming treatment of the present invention is preferably 60 mass% or less, more preferably 40 mass% or less, even more preferably 30 mass% or less, further preferably 20 mass% or less, still further preferably 15 mass% or less, and still further preferably 10 mass% or less. In addition, it is preferably 0.1 mass% or more.

The agent for hair deforming treatment of the present invention may be either a multi-part agent such as a two-part agent, or a one-part agent. From the viewpoint of ameliorating the permeability of the component (A) and the component (B) into hair and increasing the effects of the present invention, a multi-part agent comprising the component (A) and the component (B) in respective parts, and further, a two-part agent is more preferable. In the case of such a multi-part agent, a part comprising the component (B) is defined as a first agent and a part comprising the component (A) is defined as a second agent in the present invention.

In the case of the multi-part agent, the content of the component (B) in the first agent is preferably 0.2 mass% or more, more preferably 0.5 mass% or more, even more preferably 1 mass% or more, and further preferably 1.5 mass% or more, and also, it is preferably 30 mass % or less, more preferably 25 mass% or less, even more preferably 23 mass% or less, and further preferably 20 mass% or less.

In the case of the multi-part agent, the content of the component (A) in the second agent is, in terms of glyoxylic acid, preferably 2.5 mass% or more, more preferably 3 mass% or more, even more preferably 4 mass% or more, and further preferably 5 mass% or more, and also, it is preferably 30 mass% or less, more preferably 25 mass% or less, even more preferably 20 mass% or less, further preferably 15 mass% or less, and still further preferably 12 mass% or less.

When, for example, the first agent and the second agent are mixed in the multi-part agent and the obtained mixture is then used, the mixing ratio is not particularly limited. It is preferable that the composition obtained after the mixing of the parts become the agent for hair deforming treatment of the present invention. Accordingly, in the case of the multi-part agent, the contents of individual components are the contents thereof in the composition obtained after the mixing of the parts, unless otherwise specified. In addition, upon production of the multi-part agent, the multi-part agent can comprise the component (C) as a solvent for each part, and in a case where a two-part agent is produced, the component (C) is preferably present both in the part comprising the component (A) and in the part comprising the component (B).

From the viewpoint of permeability into hair, in the case of a one-part agent, the pH of the agent for hair deforming treatment of the present invention is preferably 4 or less, more preferably 3 or less, even more preferably 2.5 or less, and further preferably 2 or less. In addition, from the viewpoint of suppression of hair damage and suppression of irritation to the skin, the pH of the present agent for hair deforming treatment is preferably 1 or more, more preferably 1.2 or more, and even more preferably 1.5 or more. In the case of a multi-part agent, the pH of the part comprising the component (A), namely, the pH of the second agent is preferably set in the above described range. Moreover, in the case of a multi-part agent, from the viewpoint of preventing discoloration of the treatment agent, the pH of the part comprising the component (B), namely, the pH of the first agent is preferably 6 or less, more preferably 5 or less, and even more preferably 4.5 or less, and also, it is preferably 2.5 or more, more preferably 3 or more, and even more preferably 3.5 or more. In the present invention, the pH of the hair treatment agent indicates a value obtained by directly measuring the pH of the agent for hair deforming treatment at a room temperature (25°C), using a pH meter (manufactured by HORIBA, Model No.: F-52), without, for example, dilution of the agent for hair deforming treatment.

In order to adjust the pH of the agent for hair deforming treatment to be in the above described range, a pH adjuster can be used, as appropriate. Examples of the pH adjuster as an alkali agent that can be used include: ammonia or a salt thereof; alkanolamine such as monoethanolamine, isopropanolamine, 2-amino-2-methylpropanol or 2-aminobutanol, or a salt thereof; alkanediamine such as 1,3-propanediamine, or a salt thereof; carbonates such as guanidine carbonate, sodium carbonate, potassium carbonate, sodium hydrogen carbonate, or potassium hydrogen carbonate; and hydroxides such as sodium hydroxide or potassium hydroxide. Moreover, as an acid agent, inorganic acids such as hydrochloric acid or phosphoric acid; hydrochlorides such as monoethanolamine hydrochloride; phosphates such as monopotassium dihydrogen phosphate or disodium monohydrogen phosphate; or organic acids other than the component (A), such as lactic acid or malic acid, can be used.

From the viewpoint of improving the touch feeling of hair after completion of a hair treatment and further improving the effects of the present invention, the agent for hair deforming treatment of the present invention preferably comprises a cationic surfactant. The cationic surfactant is preferably a mono-long-chain-alkyl quaternary ammonium salt having an alkyl group containing from 8 to 24 carbon atoms and three alkyl groups containing from 1 to 4 carbon atoms.

Preferably, at least one mono-alkyl quaternary ammonium surfactant is selected from the group consisting of compounds represented by the following formula: 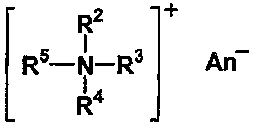 wherein R² represents a saturated or unsaturated, straight-chain or branched-chain alkyl group containing from 8 to 22 carbon atoms, R⁶-CO-NH-(CH₂)ₘ-, or R⁶-CO-O-(CH₂)ₘ- (wherein R⁶ represents a saturated or unsaturated, straight-chain or branched-chain alkyl chain containing from 7 to 21 carbon atoms, and m represents an integer of from 1 to 4); R³, R⁴ and R⁵ each independently represent an alkyl group containing from 1 to 4 carbon atoms or a hydroxylalkyl group containing from 1 to 4 carbon atoms, and An⁻ represents a chloride ion, a bromide ion, a methosulfate ion, or an ethosulfate ion.

Examples of a preferred cationic surfactant include long-chain quaternary ammonium compounds such as cetyltrimethylammonium chloride, myristyltrimethylammonium chloride, behentrimonium chloride, cetyltrimethylammonium bromide, and stearamidopropyl trimonium chloride. These compounds can be used alone, or can also be used in the form of a mixture thereof.

The content of the cationic surfactant in the agent for hair deforming treatment of the present invention is preferably 0.05 mass% or more, and more preferably 0.1 mass% or more, and also, it is preferably 10 mass% or less, and more preferably 5 mass% or less. When the agent for hair deforming treatment is a multi-part agent, the multi-part agent may comprise the cationic surfactant in the first agent, or in the second agent, or both of the first and second agents.

Furthermore, from the viewpoint of improving the touch feeling of hair after completion of a hair treatment and achieving good manageability, the agent for hair deforming treatment of the present invention preferably comprises silicone. As such silicone, dimethylpolysiloxane and amino-modified silicone are preferable.

As dimethylpolysiloxane, either a cyclic or acyclic dimethylpolysiloxane polymer can be used. Examples include SH200 series, BY22-019, BY22-020, BY11-026, B22-029, BY22-034, BY22-050A, BY22-055, BY22-060, BY22-083 and FZ-4188 (all of which are manufactured by Dow Corning Toray Co., Ltd.), and KF-9088, KM-900 series, MK-15H and MK-88 (all of which are manufactured by Shin-Etsu Chemical Co., Ltd.).

As amino-modified silicone, all silicones having an amino group or an ammonium group can be used. Examples include amino-modified silicone oil, all or a part of terminal hydroxy groups of which are sealed with, for example, methyl groups, and amodimethicone, the terminus of which is not sealed. As preferred amino-modified silicone, a compound represented by the following formula can be used, for example: 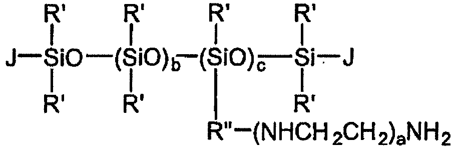 wherein R' represents a hydrogen atom, a hydroxy group, or R^{Z}, wherein R^{Z} represents a substituted or unsubstituted monovalent hydrocarbon group containing from 1 to 20 carbon atoms; J represents R^{X}, R"-(NHCH₂CH₂)ₐNH₂, OR^{Z}, or a hydroxy group, wherein R" represents a divalent hydrocarbon group containing from 1 to 8 carbon atoms; a represents a number of from 0 to 3; and b and c each represent a number, in which the sum thereof is, at a number average, 10 or more and less than 20000, preferably 20 or more and less than 3000, more preferably 30 or more and less than 1000, and even more preferably 40 or more and less than 800.

Specific examples of a commercially available product of preferred amino-modified silicone include: amino-modified silicone oils such as SF8452C and SS3551 (both of which are manufactured by Dow Corning Toray Co., Ltd.), and KF-8004, KF-867S and KF-8015 (all of which are manufactured by Shin-Etsu Chemical Co., Ltd.); and amodimethicone emulsions such as SM8704C, SM8904, BY22-079, FZ-4671 and FZ4672 (all of which are manufactured by Dow Corning Toray Co., Ltd.) .

The content of the silicone in the agent for hair deforming treatment of the present invention is preferably 0.1 mass% or more, more preferably 0.2 mass% or more, and even more preferably 0.5 mass% or more, and also, it is preferably 20 mass% or less, more preferably 10 mass% or less, and even more preferably 5 mass% or less. When the agent for hair deforming treatment is a multi-part agent, the multi-part agent may comprise the silicone in the first agent or in the second agent, or both of the first and second agents.

Further, from the viewpoint of improving the touch feeling of hair after completion of a hair treatment, the agent for hair deforming treatment of the present invention preferably comprises a cationic polymer.

The cationic polymer means a polymer having a cationic group or a group ionizable to such a cationic group. An amphoteric polymer, which can be cationic as a whole, is also included in the cationic polymer. That is to say, examples of the cationic polymer include water-soluble cationic polymers, which comprise an amino group or an ammonium group in the side chains thereof, or which comprise, as constitutional unit, a diallyl quaternary ammonium salt, such as a cationized cellulose derivative, cationic starch, a cationized guar gum derivative, a polymer or copolymer of diallyl quaternary ammonium salts, and a quaternary polyvinyl pyrrolidone derivative. In terms of effects such as touch feeling softness, smoothness, and easy to run fingers through hair obtained during rinsing or shampooing, manageability and moisture retention during drying, and the stability of the agent, among the aforementioned cationic polymers, a polymer comprising, as a constitutional unit, a diallyl quaternary ammonium salt, a quaternary polyvinyl pyrrolidone derivative, and a cationized cellulose derivative are preferable; and a polymer or copolymer of diallyl quaternary ammonium salts, and a cationized cellulose derivative are more preferable.

Specific examples of a preferred polymer or copolymer of diallyl quaternary ammonium salts include a dimethyldiallyl ammonium chloride polymer (Polyquaternium-6, for example, Marquardt 100; Lubrizol Advanced Materials), a dimethyldiallyl ammonium chloride/acrylic acid copolymer (Polyquaternium-22, for example, Marquardt 280 and Marquardt 295; Lubrizol Advanced Materials), and a dimethyldiallyl ammonium chloride/acrylamide copolymer (Polyquaternium-7, for example, Marquardt 550; Lubrizol Advanced Materials).

Specific examples of a preferred quaternary polyvinyl pyrrolidone derivative include polymers obtained by polymerizing a vinyl pyrrolidone copolymer with dimethylaminoethyl methacrylate (Polyquaternium-11, for example, GAFQUAT 734, GAFQUAT 755, and GAFQUAT 755N (all of which are manufactured by Ashland).

Specific examples of a preferred cationized cellulose include: polymers obtained by adding glycidyltrimethyl ammonium chloride to hydroxycellulose (Polyquaternium-10, for example, Leoguard G and Leoguard GP (both of which are manufactured by Lion Corporation); and Polymer JR-125, Polymer JR-400, Polymer JR-30M, Polymer LR-400, and Polymer LR-30M (all of which are manufactured by Amerchol Corp.)); and hydroxyethyl cellulose dimethyldiallyl ammonium chloride (Polyquaternium-4, for example, CELLCOAT H-100 and CELLCOAT L-200 (both of which are manufactured by Akzo Nobel)).

The content of the cationic polymer in the agent for hair deforming treatment of the present invention is preferably 0.001 mass% or more, more preferably 0.01 mass% or more, and even more preferably 0.05 mass% or more, and also, it is preferably 20 mass% or less, and more preferably 10 mass% or less. When the agent for hair deforming treatment is a multi-part agent, the multi-part agent may comprise the cationic polymer in the first agent, or in the second agent, or both of the first and second agents.

Further, the agent for hair deforming treatment of the present invention may also comprise an antioxidant in an amount ordinarily comprised in such an agent. The antioxidant may be an antioxidant generally used in the field of hair cosmetics, and an example of the antioxidant is ascorbic acid.

The agent for hair deforming treatment of the present invention may comprise, as appropriate, components generally mixed into hair cosmetics, as well as the aforementioned components. However, it is preferable that the present agent for hair deforming treatment substantially do not comprise a strong oxidizer such as hydrogen peroxide, or dihydroxyacetone.

Moreover, preferably, the agent for hair deforming treatment of the present invention substantially does not comprise a hair reducing agent. The present invention is characterized in that it makes possible to deform hair, not depending on the cleavage of the S-S bond of proteins in hair. Thus, the present invention is a technology completely different from a permanent wave agent for deforming hair by cleaving the S-S bond of proteins in hair, using a reducing agent. Examples of the hair reducing agent include thioglycolic acid, dithioglycolic acid, cysteine, thiol such as acetylcysteine, hydrogen sulfite, and a salt thereof.

In the present invention, the expression "substantially does not comprise" is used to mean that the content of a target compound in the agent for hair deforming treatment is preferably less than 0.1 mass%, more preferably less than 0.01 mass%, and even more preferably, it means that the agent for hair deforming treatment does not comprise a target compound.

Since the agent for hair deforming treatment of the present invention is highly safe to human bodies and has less damage to hair, it can be preferably used particularly for human hair.

### [Method of hair deforming treatment]

A hair treatment for semi-permanently or permanently deforming the shape of hair using the agent for hair deforming treatment of the present invention can be carried out by a method of hair treatment comprising the following steps (i) and (ii):
(i) applying the agent for hair deforming treatment of the present invention to hair, and then allowing the agent to penetrate into the hair, and
(ii) heating and shaping the hair into which the agent for hair deforming treatment has penetrated.

In the step (i), the agent for hair deforming treatment may be applied to dry hair or may also be applied to wet hair. In order to swell hair and to promote penetration of the hair treatment agent into the hair, hair is preferably wetted with water before the step (i). The mass of the agent for hair deforming treatment applied to hair in the step (i) is, at a bath ratio to the mass of the hair (the mass of the agent for hair deforming treatment / the mass of the hair), preferably 0.05 or more, more preferably 0.1 or more, even more preferably 0.25 or more, and further preferably 0.5 or more, and also, it is preferably 5 or less, more preferably 3 or less, and even more preferably 2 or less. The hair to be treated with the agent for hair deforming treatment may be either the entire hair or a part thereof.

In the step (i), the agent for hair deforming treatment can be applied to hair by any given method. In addition, when the agent for hair deforming treatment is a multi-part agent, the first agent comprising the component (B) may be mixed with the second agent comprising the component (A), and the obtained mixture may be then applied to hair. Otherwise, one of the first agent and the second agent may be applied to hair, and the other part may be then applied onto the portion applied. In a case where one part is applied onto the other part successively, from the viewpoint of promoting penetration of the agent for hair deforming treatment into hair and enhancing the effects, after one part had been applied to hair, the hair, to which the hair treatment agent has been applied, may be left, and thereafter, the other part may be applied or may also be applied onto the previously applied part. In such a case, in order to allow the agent for hair deforming treatment to penetrate and diffuse into hair, the standing time is preferably 1 minute or more, more preferably 3 minutes or more, and even more preferably 5 minutes or more, and also, it is preferably 1 hour or less, more preferably 30 minutes or less, and even more preferably 20 minutes or less. At this time, from the viewpoint of promoting penetration of the agent for hair deforming treatment into hair, heating may be carried out. When such heating is carried out, the heating is preferably carried out at a temperature of from 40°C to 90°C. Moreover, when the two parts are successively applied to hair, the order of applying the parts is not particularly limited. From the viewpoint of promoting penetration of the agent for hair deforming treatment into hair and enhancing the effects, it is more preferable that the second agent be applied to hair, after the first agent has been applied thereto.

Moreover, in the step (i), when the first agent and the second agent are successively applied to hair, after the first agent has been applied and left, a step of rinsing the first agent (hereinafter referred to as an "intermediate rinsing step") may be comprised before the application of the second agent. From the viewpoint of reduction in the treatment time, it is preferable not to comprise such an intermediate rinsing step. When the intermediate rinsing step is not comprised in the step (i), the molecular weight of the component (B) comprised in the first agent is preferably from 100 to 180, and more preferably from 100 to 140, from the viewpoint of further enhancing the hair deforming effect. On the other hand, from the viewpoint of the improvement of touch feeling after hair deformation treatment, it is preferable to establish the intermediate rinsing step. When the intermediate rinsing step is comprised in the step (i), the molecular weight of the component (B) comprised in the first agent is preferably 140 to 1000, and more preferably from 180 to 1000, from the viewpoint of further enhancing the hair deforming effect and improving the touch feeling after hair deformation.

Furthermore, when the two parts are successively applied to hair, the amounts of the first agent and the second agent applied are not particularly limited. The two parts are applied, such that the molar ratio of the amount of the component (B) in the first agent applied to hair to the amount of the component (A) in the second agent applied to hair, (B)/(A), can be preferably 0.01 or more, more preferably 0.1 or more, even more preferably 0.3 or more, and further preferably 0.5 or more, and also, it is preferably 5 or less, more preferably 4 or less, even more preferably 3 or less, further preferably 2 or less, and still further preferably 1.5 or less. Specifically, for example, the ratio of the amount of the component (B) applied to hair, which is calculated from the content of the component (B) in the first agent and the amount of the first agent applied onto the hair, and the amount of the component (A) applied to hair, which is calculated from the content of the component (A) in the second agent and the amount of the second agent applied onto the hair, may be within the aforementioned range at a molar ratio.

A step of leaving hair, to which the hair treatment agent has been applied, may be inserted between the step (i) and the step (ii). In such a case, in order to allow the agent for hair deforming treatment to penetrate and diffuse into hair, the standing time is preferably 1 minute or more, more preferably 3 minutes or more, and even more preferably 5 minutes or more, and also, it is preferably 1 hour or less, more preferably 30 minutes or less, and even more preferably 20 minutes or less.

Moreover, from the viewpoint of promoting penetration of the agent for hair deforming treatment into hair, in the step of leaving hair, the hair may be heated. When the hair is heated, it is preferable to heat it at a temperature of from 40°C to 90°C. By this heating operation, since a low-order oligomer can be produced in hair before the step (ii), heating is preferable, also in that the step (ii) can be carried out more advantageously.

Between the step (i) and the step (ii), the hair, to which the hair treatment agent has been applied, may be rinsed, or may not be rinsed. From the viewpoint of enhancing the effect of sufficiently retaining the components of the hair treatment agent in hair, giving a semi-permanent shape to the hair, and then semi-permanently deforming the shape of hair again by heating, it is preferable not to rinse the hair between the step (i) and the step (ii) .

In order to increase interaction between the components (A) and (B) and hair proteins, and also in order to promote a condensation reaction between the component (A) and the component (B) in hair to obtain the effects of the present invention, the heating temperature in the step (ii) is preferably 50°C or higher, more preferably 60°C or higher, and even more preferably 80°C or higher, and also, in order to suppress rapid evaporation of water during heating, the heating temperature is preferably 250°C or lower, more preferably 240°C or lower, and even more preferably 230°C or lower. Examples of the heating method include methods using a hair iron, an electric heating rod, a hot curler, etc.

The heating time applied in the step (ii) is selected, as appropriate, depending on the heating device and/or the heating temperature used. From the viewpoint of allowing the agent for hair deforming treatment to penetrate and diffuse into hair and to promote sufficient polymerization, the heating time is 1 second or more, preferably 5 seconds or more, more preferably 1 minute or more, even more preferably 5 minutes or more, further preferably 15 minutes or more, and still further preferably 30 minutes or more, and also, in order to suppress hair damage, it is preferably 2 hours or less, more preferably 1 hour or less, and even more preferably 45 minutes or less.

Imparting a hair shape in the step (ii) includes both the imparting a straight shape and the imparting a curly shape. Examples of the method of giving a straight shape to hair include a method of blow-drying hair while pulling the hair with a hand, or a tool such as a comb or a brush, and a method of heating hair using a hair iron. From the viewpoint of the ease of deformation, the method of using a hair iron is preferable. In order to provide a straight shape to hair, while heating the hair using a hair iron, a method of holding hair with a flat iron and then moving the flat iron from the roots to the tips, or a method of holding hair with a flat iron and then retaining it, while pulling the hair with a hand, or a tool such as a comb or a brush, may be applied. Otherwise, a combination of the two above methods may also be applied. When a curly shape is given to hair, for example, a method of winding hair around an electric heating rod, a hot curler, etc., and then retaining it, while heating the hair, a method of winding hair around a curl iron and then retaining it, are applied.

The step (ii) is preferably carried out in an environment in which rapid evaporation of water is suppressed. Examples of a specific means for suppressing evaporation of water include a method of coating hair, to which the hair treatment agent has been applied, with a plastic film such as plastic film for food products, a cap, etc., and a method of continuously spraying water vapor such as superheated vapor to hair.

After completion of the step (ii), hair may be rinsed, or may not be rinsed. From the viewpoint of preventing reduction in hair touch feeling due to redundant polymers, the hair is preferably rinsed.

It is considered that, by these treatments, the components (A) and (B) penetrate into hair, and thereby, an interaction of these components with hair proteins occurs. In addition, it is considered that, in the hair, a thermoplastic condensate of the components (A) and (B) is generated. Thus, the shape of hair can be easily deformed by heating hair, and further, once the treatment is performed, hair can be freely and repeatedly deformed, semi-permanently or permanently, only by heating the hair, without applying the hair treatment agent again. Further, hair deformation provided by the method of the present invention is not lost, even if, for example, the hair is repeatedly washed with shampoo.

### (Re-deforming treatment method)

After hair has been subjected to deforming treatment by a method comprising the step (i) or the step (ii), a step of semi-permanently re-deforming the hair to another shape by heating can be carried out. The temperature of the heat given for re-deformation is preferably 30°C or higher, and more preferably 40°C or higher, and also, it is preferably 230°C or lower, more preferably 220°C or lower, and even more preferably 210°C or lower. In addition, when hair is subjected to re-deforming treatment, it is preferable not to apply any one of the agent for hair deforming treatment of the present invention, a hair treatment agent comprising a reducing agent, such as, what is called, a permanent wave agent, and known agent for hair deforming treatments such as an alkaline relaxer.

Hereafter, specific procedures for carrying out a step of semi-permanently re-deforming hair to another shape by heating will be described.

### • Case of re-deforming hair already curly shaped into straight shape

In order to re-deform hair, which has been once subjected to deforming treatment into a curly shape, into a straight shape, for example, a method of blow-drying hair with a dryer, while pulling the hair with a hand, or a tool such as a comb or a brush, a method of heating hair with a hair iron, are applied. From the viewpoint of the ease of deformation, the method of using the hair iron is preferable. In order to provide a straight shape to hair, while heating the hair with a hair iron, a method of holding hair with a hair iron and then moving the hair iron from the roots to the tips, or a method of holding hair with a hair iron and then retaining it, while pulling the hair with a tool such as a comb or a brush, may be applied. Otherwise, a combination of the two above methods may also be applied.

In this case, from the viewpoint of deforming the shape of hair semi-permanently or permanently, regardless of the type of a hair iron used, the material of a heating portion, a preset temperature, and the operational method of an iron, the attained temperature during the heating of hair (the temperature of hair) is preferably 120°C or higher, and more preferably 150°C or higher, and also, from the viewpoint of achieving both prevention of hair damage and semi-permanent or permanent deformation of the shape of hair, the attained temperature is preferably 230°C or lower, more preferably 220°C or lower, and even more preferably 210°C or lower. The temperature at which hair is heated can be measured, for example, using a radiation thermometer (Model No.: ST653) manufactured by SENTRY.

### • Case of re-deforming hair already straightly shaped into curly shape

In order to re-deform hair, which has been once subjected to deforming treatment into a straight shape, into a curly shape, for example, a method of winding hair around a rod, a curler, etc., and then retaining it, while heating the hair, a method of winding hair around a hair iron and then retaining it, are applied.

In this case, from the viewpoint of deforming the shape of hair semi-permanently or permanently, the attained temperature during the heating of hair (the temperature of hair) is preferably 30°C or higher, and more preferably 40°C or higher, and also, from the viewpoint of achieving both prevention of hair damage and semi-permanent or permanent deformation of the shape of hair, the' attained temperature is preferably 180°C or lower, more preferably 120°C or lower, even more preferably 100°C or lower, further preferably 80°C or lower, and still further preferably 60°C or lower.

Even in a case where hair is re-deformed, upon heating it, either a method of heating hair, which is dried, or a method of heating hair after wetting it with water, may be applied. From the viewpoint of semi-permanently or permanently deforming the shape of hair, the method of heating hair after wetting it with water is preferable.

The heating time applied when hair is re-deformed is selected, as appropriate, depending on a heating device used and a heating temperature. From the viewpoint of semi-permanently or permanently deforming the shape of hair, the heating time is preferably 1 second or more, more preferably 5 seconds or more, even more preferably 1 minute or more, further preferably 5 minutes or more, still further preferably 15 minutes or more, and still further preferably 30 minutes or more. In addition, in order to suppress hair damage, it is preferably 2 hours or less, more preferably 1 hour or less, and even more preferably 45 minutes or less.

Preferred method of hair treatments, which semi-permanently or permanently deform hair, include the following three patterns.
Pattern 1: Case where the hair treatment agent is a one-part agent
   1) Hair is optionally wetted with water.
   2) The hair treatment agent of the present invention, which comprises the following components (A), (B) and (C), wherein the molar ratio of the content of the component (B) to the content of the component (A) is from 0.01 to 5, is applied to hair, and it is then allowed to penetrate into it:
      Component (A): glyoxylic acid or a hydrate or salt thereof,
      Component (B): one or two or more selected from the group consisting of catechin, epigallocatechin, epigallocatechin gallate, naringenin, equol, and a green tea extract, and
      Component (C): water.
   3) The hair, to which the hair treatment agent has been applied, is optionally left for 1 minute or more and 1 hour or less. During this operation, the hair may be optionally heated to a temperature of from 40°C to 90°C.
   4) The hair is heated and shaped at a temperature of from 50°C to 250°C.
   5) The hair is optionally rinsed.
   6) The hair is optionally heated at a temperature of from 40°C to 230°C and re-deformed.
Pattern 2: Case where the hair treatment agent is a two-part agent -1
   1) Hair is optionally wetted with water.
   2) The hair treatment agent of the present invention, in which a first agent comprising the following components (B) and (C) is mixed with a second agent comprising the following components (A) and (C), so that the molar ratio of the content of the component (B) to the content of the component (A) can be from 0.01 to 5, is applied to hair, and it is then allowed to penetrate into it:
      Component (A): glyoxylic acid or a hydrate or salt thereof,
      Component (B): one or two or more selected from the group consisting of catechin, epigallocatechin, epigallocatechin gallate, naringenin, equol, and a green tea extract, and
      Component (C): water.
   3) The hair, to which the hair treatment agent has been applied, is optionally left for 1 minute or more and 1 hour or less. During this operation, the hair may be optionally heated to a temperature of from 40°C to 90°C.
   4) The hair is heated and shaped at a temperature of from 50°C to 250°C.
   5) The hair is optionally rinsed.
   6) The hair is optionally heated at a temperature of from 40°C to 230°C to re-deform it.
Pattern 3: Case where the hair treatment agent is a two-part agent -2
   1) Hair is optionally wetted with water.
   2) A first agent comprising the following components (B) and (C) is applied to hair, and it is then allowed to penetrate into it:
      Component (B): one or two or more selected from the group consisting of catechin, epigallocatechin, epigallocatechin gallate, naringenin, equol, and a green tea extract, and
      Component (C): water.
   3) The hair is optionally left for 1 minute or more and 1 hour or less. During this operation, the hair may be optionally heated to a temperature of from 40°C to 90°C.
   4) The first agent is optionally washed away from the hair.
   5) The second agent comprising the following components (A) and (C) is applied onto the portion in hair, to which the first agent has been applied, so that the molar ratio of the content of the component (B) to the content of the component (A) can be 0.01 to 5, and it is then allowed to penetrate into the hair:
      Component (A): glyoxylic acid or a hydrate or salt thereof, and
      Component (C): water.
   6) The hair is optionally left for 1 minute or more and 1 hour or less. At this time, the hair is optionally heated to a temperature of from 40°C to 90°C.
   7) The hair is heated and shaped at a temperature of from 50°C to 250°C.
   8) The hair is optionally rinsed.
   9) The hair is optionally heated at a temperature of from 40°C to 230°C and re-deformed.

Since the method of hair treatment of the present invention is a technology capable of freely changing hair based on a principle, which is completely different from a permanent wave treatment using a reducing agent or a relaxer treatment using a strongly-alkaline hair treatment agent having pH 12 to 14, the present method does not comprise a step of applying a hair treatment agent comprising a reducing agent or a strongly-alkaline hair treatment agent having pH 12 to 14 to the hair. Accordingly, it can be said that, in comparison to the aforementioned conventional hair deformation methods, the method of hair treatment of the present invention is also advantageous in that hair can be deformed without being damaged.

With regard to the aforementioned embodiments, preferred aspects of the present invention will be further disclosed below.
<1> An agent for hair deforming treatment comprising the following components (A), (B) and (C):
   (A) glyoxylic acid or a hydrate or salt thereof,
   (B) one or two or more selected from the group consisting of compounds represented by the following formula (1): 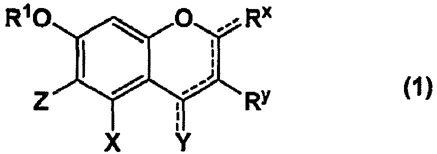 wherein
      R¹ represents a hydrogen atom or a methyl group,
      X represents a hydrogen atom, a hydroxy group, or a methoxy group,
      Y represents a hydrogen atom, an oxygen atom, a hydroxy group, or a methoxy group,
      Z represents a hydrogen atom, or a straight-chain or branched-chain alkyl group or alkenyl group containing from 1 to 5 carbon atoms,
      R^{x} represents a hydrogen atom, an oxygen atom, a hydroxy group, a methoxy group, or an aromatic hydrocarbon group, which is optionally substituted with up to three hydroxy groups or methoxy groups and which optionally forms a condensed ring with 1,3-dioxolane,
      R^{y} represents a hydrogen atom, a hydroxy group, a methoxy group, or an aromatic hydrocarbon group, which is optionally substituted with up to three hydroxy groups or methoxy groups and which optionally forms a condensed ring with 1,3-dioxolane, or an arylcarbonyloxy group or aralkylcarbonyloxy group, which is optionally substituted with up to three hydroxy groups or methoxy groups, and
      the dashed line optionally represents a double bond,
      provided that the dashed line and the solid line, which are adjacent to R^{x} or Y, each represent a double bond only when R^{x} or Y is an oxygen atom, and otherwise these lines each represent a single bond, and provided that,
      Z represents a straight-chain or branched-chain alkyl group or alkenyl group containing from 1 to 5 carbon atoms, only when R^{x} or R^{y} is an o,p-dihydroxy aromatic hydrocarbon group, and otherwise Z is a hydrogen atom, and
   (C) water,
   wherein the molar ratio of the content of the component (B) to the content of the component (A), (B)/(A), is 0.1 or more and 5 or less.
<2> The agent for hair deforming treatment according to <1> above, wherein the molar ratio of the content of the component (B) to the content of the component (A), (B)/(A), is preferably 0.02 or more, more preferably 0.1 or more, even more preferably 0.3 or more, and further preferably 0.5 or more, and also, it is preferably 4 or less, more preferably 3 or less, even more preferably 2 or less, further preferably 1.5 or less, still further preferably 1 or less, and still further preferably 0.6 or less.
<3> The agent for hair deforming treatment according to <1> or <2> above, wherein the content of the component (A) is, relative to the content of glyoxylic acid, preferably 2.5 mass% or more, more preferably 3 mass% or more, even more preferably 4 mass% or more, and further preferably 5 mass% or more, and also, it is preferably 30 mass% or less, more preferably 25 mass% or less, even more preferably 20 mass% or less, further preferably 15 mass% or less, and still further preferably 12 mass% or less.
<4> The agent for hair deforming treatment according to any one of <1> to <3>, wherein the content of the component (B) is preferably 0.2 mass% or more, more preferably 0.5 mass% or more, even more preferably 1 mass% or more, and further preferably 1.5 mass% or more, and also, it is preferably 30 mass% or less, more preferably 25 mass% or less, even more preferably 23 mass% or less, and further preferably 20 mass% or less.
<5> The agent for hair deforming treatment according to any one of <1> to <4>, wherein the pH thereof is preferably 4 or less, more preferably 3 or less, even more preferably 2.5 or less, and further preferably 2 or less, and also, it is preferably 1 or more, more preferably 1.2 or more, and even more preferably 1.5 or more.
<6> The agent for hair deforming treatment according to any one of <1> to <5>, wherein the molecular weight of the component (B) is preferably 150 or more, and also, it is preferably 1000 or less, more preferably 700 or less, and even more preferably 500 or less.
<7> The agent for hair deforming treatment according to any one of <1> to <6>, wherein the component (B) is preferably one or two or more selected from the group consisting of flavan-3-ol compounds represented by the following formula (1-1-A), flavonol compounds represented by the following formula (1-3-B), flavanone compounds represented by the following formula (1-2-A), flavone compounds represented by the following formula (1-3-A), isoflavone compounds represented by the following formula (1-4-A), isoflavane compounds represented by the following formula (1-4-B), and coumarin compounds represented by the following formula (1-5): 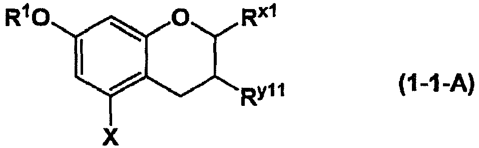 wherein R¹ and X are as defined above, R^{x1} represents an aromatic hydrocarbon group, which is optionally substituted with up to three hydroxy groups or methoxy groups and which optionally forms a condensed ring with 1,3-dioxolane, R^{y11} represents a hydroxy group, a methoxy group, or an aromatic hydrocarbon group, which is optionally substituted with up to three hydroxy groups or methoxy groups and which optionally forms a condensed ring with 1,3-dioxolane, or an arylcarbonyloxy group or aralkylcarbonyloxy group, which is optionally substituted with up to three hydroxy groups or methoxy groups, 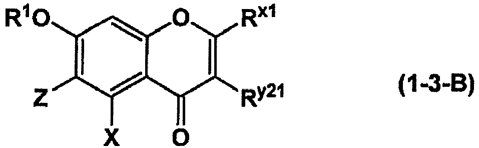
   wherein R¹, X, Z and R^{x1} are as defined above, and R^{y21} represents a hydroxy group or a methoxy group,
   provided that Z represents a straight-chain or branched-chain alkyl group or alkenyl group containing from 1 to 5 carbon atoms, only when R^{x1} is an o,p-dihydroxy aromatic hydrocarbon group, and otherwise Z is a hydrogen atom,

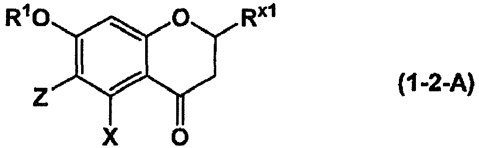

   wherein R¹, X, Z and R^{x1} are as defined above,
   provided that Z represents a straight-chain or branched-chain alkyl group or alkenyl group containing from 1 to 5 carbon atoms, only when R^{x1} is an o,p-dihydroxy aromatic hydrocarbon group, and otherwise Z is a hydrogen atom,

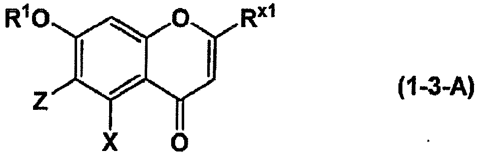

   wherein R¹, X, Z and R^{x1} are as defined above,
   provided that Z represents a straight-chain or branched-chain alkyl group or alkenyl group containing from 1 to 5 carbon atoms, only when R^{x1} is an o,p-dihydroxy aromatic hydrocarbon group, and otherwise Z is a hydrogen atom,

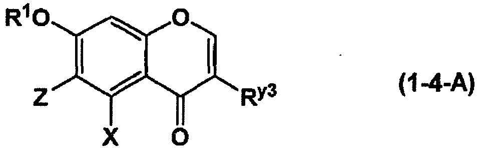

   wherein R¹, X and Z are as defined above, R^{y3} represents an aromatic hydrocarbon group, which is optionally substituted with up to three hydroxy groups or methoxy groups and which optionally forms a condensed ring with 1,3-dioxolane,
   provided that Z represents a straight-chain or branched-chain alkyl group or alkenyl group containing from 1 to 5 carbon atoms, only when R^{y3} is an o,p-dihydroxy aromatic hydrocarbon group, and otherwise Z is a hydrogen atom, 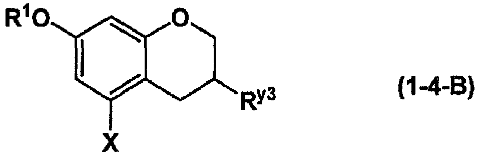
   wherein R¹, X and R^{y3} are as defined above, and 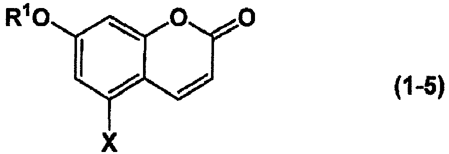 wherein R¹ and X are as defined above.
<8> The agent for hair deforming treatment according to any one of <1> to <7>, wherein the component (B) is preferably one or two or more selected from the group consisting of catechin, epicatechin, epigallocatechin, catechin gallate, epicatechin gallate, epigallocatechin gallate, quercetin, morin, hesperetin, naringenin, chrysin, daidzein, equol, umbeliiferone, and a green tea extract; more preferably one or two or more selected from the group consisting of catechin, epigallocatechin, epigallocatechin gallate, naringenin, and equol; and even more preferably one or two or more selected from the group consisting of catechin, epigallocatechin, epigallocatechin gallate, naringenin, and equol.
<9> The agent for hair deforming treatment according to any one of <1> to <8>, wherein, preferably, deformation of hair is not caused by the cleavage and recombination of the S-S bond of hair proteins.
<10> The agent for hair deforming treatment according to any one of <1> to <9>, wherein the total amount of components for reducing proteins in hair is preferably less than 0.1 mass%, and more preferably less than 0.01 mass, and even more preferably, the agent for hair deforming treatment does not comprise the ingredient therein.
<11> The agent for hair deforming treatment according to <10>, wherein the components for reducing proteins in hair are thiol, hydrogen sulfite, and salts thereof.
<12> The agent for hair deforming treatment according to <11>, wherein the thiol is preferably thioglycolic acid, dithioglycolic acid, cysteine, or acetylcysteine.
<13> The agent for hair deforming treatment according to any one of <1> to <12>, wherein the contents of hydrogen peroxide and dihydroxyacetone are each preferably less than 0.1 mass%, more preferably less than 0.01 mass%, and even more preferably, the agent for hair deforming treatment does not comprise the aforementioned compounds.
<14> The agent for hair deforming treatment according to any one of <1> to <13>, which is preferably a multi-part agent comprising a first agent comprising the component (B) and a second agent comprising the component (A).
<15> The agent for hair deforming treatment according to <14>, wherein the pH of the second agent is preferably 4 or less, more preferably 3 or less, even more preferably 2.5 or less, and further preferably 2 or less, and also, it is preferably 1 or more, more preferably 1.2 or more, and even more preferably 1.5 or more.
<16> The agent for hair deforming treatment according to <14> or <15>, wherein the content of the component (A) in the second agent is, in terms of glyoxylic acid, preferably 2.5 mass% or more, more preferably 3 mass% or more, even more preferably 4 mass% or more, and further preferably 5 mass% or more, and also, it is preferably 30 mass% or less, more preferably 25 mass% or less, even more preferably 20 mass % or less, further preferably 15 mass% or less, and still further preferably 12 mass% or less.
<17> The agent for hair deforming treatment according to any one of <14> to <16>, wherein the content of the component (B) in the first agent is preferably 0.2 mass% or more, more preferably 0.5 mass% or more, even more preferably 1 mass% or more, and further preferably 1.5 mass% or more, and also, it is preferably 30 mass% or less, more preferably 25 mass% or less, even more preferably 23 mass% or less, and further preferably 20 mass% or less.
<18> The multi-part agent for hair deforming treatment according to any one of <14> to <17>, wherein the composition after completion of the mixing of the components is the composition according to any one of <1> to <13>.
<19> A method of hair treatment for semi-permanently or permanently deforming the shape of hair, which comprises the following steps (i) and (ii):
   (i) applying the agent for hair deforming treatment according to any one of <1> to <18> to hair, and then allowing the agent to penetrate into the hair, and
   (ii) heating and shaping the hair, into which the agent for hair deforming treatment has penetrated.
<20> The method of hair treatment according to <19>, wherein the step (i) is preferably a step of mixing a first agent of the multi-part agent for hair deforming treatment according to <14> to <18> with a second agent thereof, and then applying the obtained mixture to hair.
<21> The method of hair treatment according to <19>, wherein the step (i) is preferably a step of applying one of the first agent and the second agent of the multi-part agent for hair deforming treatment according to <14> to <18> to hair, and then applying the other part onto the portion applied.
<22> The method of hair treatment according to <19>, wherein the step (i) is preferably a step of applying the first agent comprising the component (B), and then applying the second agent comprising the component (A) onto the portion applied.
<23> The method of hair treatment according to <21> or <22>, wherein the amounts of the first agent and the second agent, when they are successively applied, are amounts, such that the molar ratio of the component (B) to the component (A) on hair, (B)/(A), can be preferably 0.01 or more, more preferably 0.1 or more, even more preferably 0.3 or more, and further preferably 0.5 or more, and also, it can be preferably 5 or less, more preferably 4 or less, even more preferably 3 or less, further preferably 2 or less, and still further preferably 1.5 or less.
<24> The method of hair treatment according to any one of <21> to <23>, which preferably comprises a step of leaving hair, to which one of the first agent and the second agent has been applied, between the step of applying the one part to the hair and the step of applying the other part onto the portion applied.
<25> The method of hair treatment according to <24>, wherein when the hair to which one part has been applied is left, the hair is left, preferably while being heated at a temperature of from 40°C to 90°C.
<26> The method of hair treatment according to any one of <19> to <25>, which preferably comprises a step of wetting hair before the step (i).
<27> The method of hair treatment according to any one of <19> to <26>, wherein the heating temperature in the step (ii) is preferably 50°C or higher, more preferably 60°C or higher, and even more preferably 80°C or higher, and also, it is preferably 250°C or lower, more preferably 240°C or lower, and even more preferably 230°C or lower.
<28> The method of hair treatment according to any one of <19> to <27>, wherein the step (ii) is preferably carried out under an environment in which evaporation of water is suppressed.
<29> The method of hair treatment according to any one of <19> to <28>, which preferably does not comprise a step of applying a hair treatment agent comprising a reducing agent or a strongly-alkaline hair treatment agent having pH 12 to 14 to the hair.
<30> The method of hair treatment according to any one of <19> to <29>, wherein the mass of the agent for hair deforming treatment applied to hair in the step (i) is, at a bath ratio of the mass of the agent for hair deforming treatment to the mass of the hair (the mass of the agent for hair deforming treatment / the mass of the hair), preferably 0.05 or more, more preferably 0.1 or more, even more preferably 0.25 or more, and further preferably 0.5 or more, and also, it is preferably 5 or less, more preferably 3 or less, and even more preferably 2 or less.
<31> The method of hair treatment according to any one of <19> to <30>, wherein the heating time in the step (ii) is preferably 1 second or more, more preferably 5 seconds or more, even more preferably 1 minute or more, further preferably 5 minutes or more, still further preferably 15 minutes or more, and still further preferably 30 minutes or more, and also, it is preferably 2 hours or less, more preferably 1 hour or less, and even more preferably 45 minutes or less.
<32> The method of hair treatment according to any one of <19> to <31>, which preferably comprises a step of leaving hair, to which the hair treatment agent has been applied, between the step (i) and the step (ii).
<33> The method of hair treatment according to <32>, wherein the leaving time is preferably 1 minute or more, more preferably 3 minutes or more, and even more preferably 5 minutes or more, and also, it is preferably 1 hour or less, more preferably 30 minutes or less, and even more preferably 20 minutes or less.
<34> The method of hair treatment according to <32> or <33>, wherein hair is preferably heated at a temperature of from 40°C to 90°C during the leaving time between the step (i) and the step (ii).
<35> The method of hair treatment according to any one of <19> to <34>, which preferably does not comprise a step of rinsing hair, to which the hair treatment agent has been applied, between the step (i) and the step (ii).
<36> The method of hair treatment according to any one of <19> to <35>, which preferably comprises a step of rinsing hair after the step (ii).
<37> The method of hair treatment according to any one of <19> to <36>, wherein hair is preferably re-deformed into a different shape by heating, after the step (ii).
<38> The method of hair treatment according to <37>, wherein preferably the agent for hair deforming treatment is not applied, when hair is re-deformed.
<39> The method of hair treatment according to <37> or <38>, wherein, preferably, the heating temperature when hair is re-deformed is preferably 30°C or higher, and more preferably 40°C or higher, and also it is preferably 230°C or lower, more preferably 220°C or lower, and even more preferably 210°C or lower.
<40> Use of the composition according to any one of <1> to <18> for semi-permanent or permanent hair deformation.

### [Examples]

### Examples 1 to 12 and Comparative Examples 1 to 4

The treatment agents of a two-part agent shown in Table 1 were prepared, and the following three-step hair treatment processes were then carried out using the agents. The shape-giving effect of each agent at each step was evaluated. The results are also shown in Table 1. It is to be noted that the pH of each composition was measured by leaving the prepared composition at a room temperature (25°C) and then measuring the pH with a pH meter (manufactured by HORIBA / Model No.: F-52).

### <I: Imparting a semi-permanent curly shape>

1. A 25 cm-long tress, which consisted of 0.5 g of Caucasian straight hair, was wetted with tap water at 30°C for 30 seconds, and the wet tress was then wound around a plastic rod with a diameter of 14 mm, and fixed with a clip.
2. 1 g of the formulated first agent was applied to the tress wound around the rod, and the rod was then entirely covered with a plastic film for hermetical sealing. The tress was then heated for 1 hour in an oven, which was set at 90°C.
3. The tress was removed from the oven, and cooled to a room temperature.
4. 1 g of the formulated second agent was applied to the tress wound around the rod. The rod was entirely covered with a plastic film for hermetical sealing, and it was then heated for 1 hour in an oven, which was set at 90°C.
5. The tress was removed from the oven, and cooled to a room temperature.
6. The tress was removed from the rod, and was then rinsed with running tap water at 30°C for 30 seconds. Thereafter, a shampoo for evaluation was lathered on the tress for 60 seconds.
7. The tress was rinsed with running tap water at 30°C for 30 seconds, and immersed at an infinite bath ratio in tap water at 30°C for 60 seconds. Thereafter, the tress was gently pulled up out of the water while the root thereof being held, and water was then drained off by light shaking.
8. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair has been maintained, and the hair shape has not changed from before the treatment.

### <II: Imparting a semi-permanent straight shape to semi-permanent curly-shaped hair>

1. The tress, which had been evaluated in <I: Imparting a semi-permanent curly shape> above, was detangled by combing, and a flat iron with an actual temperature of 180°C was slid through the tress at a rate of 5 cm/sec six times.
2. The tress was rinsed with running tap water at 30°C for 30 seconds, and a shampoo for evaluation was lathered on the tress for 60 seconds. Thereafter, the tress was rinsed with running tap water at 30°C for 30 seconds, and it was then dried with a towel.
3. The tress was dried, while shaking it, so that the natural shape of hair could appear (wherein dryer was not used), and it was then combed. Thereafter, the tress was hung, and was then observed from the side by visual observation.

### (Evaluation criteria)

A: The curl has not remained, and the hair is completely deformed into straight hair.
B: The curl has become weaker than before the treatment with a flat iron, but the hair has not been completely deformed into straight hair.
C: The curly hair has been maintained, and the hair shape has not changed from before the treatment.

### <III: Imparting a semi-permanent curly shape to semi-permanent straight-shaped hair>

1. The tress, which had been evaluated in <II: Imparting a semi-permanent straight shape to semi-permanent curly-shaped hair> above, was wetted with tap water at 30°C for 30 seconds, and the wet tress was then wound around a plastic rod with a diameter of 14 mm, and fixed with a clip.
2. The rod was entirely covered with a plastic film for hermetical sealing. Thereafter, the tress was heated for 1 hour in an oven, which was set at 40°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and immersed at an infinite bath ratio in tap water at 30°C for 60 seconds. Thereafter, the tress was gently pulled up out of the water while the root thereof being held, and water was then drained off by light shaking.
6. The tress was hung and left allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair has been maintained, and the hair shape has not changed from before the treatment.

### <Formulation of shampoo for evaluation>

| Component | (mass%) |
|---|---|
| Sodium laureth sulfate | 15.5 |
| Lauramide DEA | 1.5 |
| Sodium benzoate | 0.5 |
| EDTA-2Na | 0.3 |
| Phosphoric acid | Amount for adjusting to pH7 |
| Deionized water | balance |
| Total | 100 |

**[Table 1]**

| | | | | | Example | | | | | | | | | | | | Comparative Example | | | |
|---|---|---|---|---|---|---|---|---|---|---|---|---|---|---|---|---|---|---|---|---|
| | | | | | 1 | 2 | 3 | 4 | 5 | 6 | 7 | 8 | 9 | 10 | 11 | 12 | 1 | 2 | 3 | 4 |
| Formulated first agent | Formulation [mass%] | | | (+)-Catechin | 0.5 | 1.5 | 5 | 9.9 | 19.8 | - | - | 9.9 | - | - | 9.9 | 9.9 | 0.1 | - | 19.8 | - |
| | | | | (-)-Epigallocatechin | - | - | - | - | - | 10.4 | - | - | - | - | - | - | - | - | - | - |
| | | | (B) | (-)-Epigailocatechin gallate | - | - | - | - | - | - | 15.6 | - | - | - | - | - | - | - | - | - |
| | | | | Naringenin | - | - | - | - | - | - | - | - | 4.6 | - | - | - | - | - | - | - |
| | | | | Equol | - | - | - | - | - | - | - | - | - | 8.3 | - | - | - | - | - | - |
| | | | (B') | Tannic acid | - | - | - | - | - | - | - | - | - | - | - | - | - | 23.2 | - | - |
| | | | Others | Ethanol | 40 | 40 | 40 | 40 | 40 | 40 | 40 | 80 | 80 | 80 | 40 | 40 | 40 | - | 40 | - |
| | | | | Sodium hydroxide | (*1) | | | | | | | | | | | | | | | |
| | | | (C) | Deionized water | Balance | | | | | | | | | | | | | | | |
| | | | Total | | 100 | | | | | | | | | | | | | | | |
| | pH | | | | 4 | | | | | | | | | | | | | | | |
| Formulated second agent | Formulation [mass%] | | (A) | Glyoxylic acid | 10 | 10 | 10 | 10 | 10 | 10 | 10 | 10 | 10 | 10 | 5 | 20 | 10 | 10 | 1 | 20 |
| | | | Others | Sodium hydroxide | (*2) | | | | | | | | | | | | | | | |
| | | | (C) | Deionized water | Balance | | | | | | | | | | | | | | | |
| | | | Total | | 100 | | | | | | | | | | | | | | | |
| | pH | | | | 2 | | | | | | | | | | | | | | | |
| Component molar ratio | | (B)/(A) or (B')/(A) | | | 0.013 | 0.038 | 0.13 | 0.25 | 0.51 | 0.25 | 0.25 | 0.25 | 0.13 | 0.25 | 0.5 | 0.125 | 0.003 | 0.1 | 5.1 | - |
| Evaluation results | | I. Imparting a semi-permanent curly shape | | | D | C | B | A | A | A | A | A | B | D | A | A | E | E | E | E |
| | | II. Imparting a semi-permanent straight shape to semi-permanent curly-shaped hair | | | A | A | A | A | A | A | A | A | A | A | A | A | *3 | *3 | *3 | *3 |
| | | III. Imparting a semi-permanent curly shape to semi-permanent straight-shaped hair | | | C | C | C | 8 | B | B | B | B | C | D | C | B | *3 | *3 | *3 | *3 |

| | | | | | | | | | | | | | | | | | | | | |
|---|---|---|---|---|---|---|---|---|---|---|---|---|---|---|---|---|---|---|---|---|
| *1: Amount for adjusting to pH 4.0 *2: Amount for adjusting to pH 2.0 *3: Since curly shape could not be given to hair in Evaluation I, not evaluated. | | | | | | | | | | | | | | | | | | | | |

### Example 13

The treatment agents shown in Table 2 were prepared, and the following three-step hair treatment process was then carried out using the agents. The shape-giving effect of each agent at each step was evaluated. The results are also shown in Table 2.

### <I: Imparting a semi-permanent straight shape>

1. A tress consisting of slightly spread, straight hair from Caucasian race (untreated hair) (weight: 0.5 g / length: 25 cm) was wetted with tap water at 30°C for 30 seconds, and the wet tress was then wound around a plastic rod with a diameter of 14 mm, and fixed with a clip.
2. 1.0 g of the formulated first agent was applied to the tress wound around the rod, and the rod was entirely covered with a plastic film for hermetical sealing, and heated for 1 hour in an oven, which was set at 90°C.
3. The tress was removed from the oven, and cooled to a room temperature.
4. The plastic film was uncovered, and water was lightly removed with a towel.
5. 1.0 g of the formulated second agent was applied to the tress wound around the rod, and the rod was entirely covered with a plastic film for hermetical sealing, and heated for 1 hour in an oven, which was set at 40°C.
6. The tress was removed from the oven, and cooled to a room temperature.
7. The plastic film was uncovered, the tress was removed from the rod, and water was lightly removed with a towel. Thereafter, the tress was dried with hot air from a dryer, until it was completely dried.
8. The tress was combed to detangle it, and a flat iron with an actual temperature of 230°C was slid through the tress at a rate of 5 cm/sec six times, so that the style of straight hair was completely formed from the roots to the tips of the tress.
9 The tress was rinsed with running tap water at 30°C for 30 seconds. Thereafter, a shampoo for evaluation was lathered on the tress for 60 seconds. After that, the tress was rinsed with running tap water at 30°C for 30 seconds, and was then dried with a towel.
10. The tress was dried, while being shaken, so that the shape of hair could directly appear, and it was then combed. Thereafter, the tress was hung, and was then visually observed from the side.

### (Evaluation criteria)

A: The spreading of the tress disappears, and completely straight hair is maintained from the roots to the tips of the tress.
B: Although the tress is straight hair, in which the spreading of the tress is suppressed, it is slightly spread.
C: The spreading of the tress is equivalent to that of untreated hair.

### <II: Imparting a semi-permanent curly shape to semi-permanent straight-shaped hair>

1. The tress, which had been evaluated in <I: Imparting a semi-permanent straight shape> above, was wetted with tap water at 30°C for 30 seconds, and the wet tress was then wound around a plastic rod with a diameter of 14 mm, and fixed with a clip.
2. The rod was entirely covered with a plastic film for hermetical sealing. Thereafter, the tress was heated for 1 hour in an oven, which was set at 40°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and immersed at an infinite bath ratio in tap water at 30°C for 60 seconds. Thereafter, the tress was gently pulled up out of the water while the root thereof being held, and water was then drained off by light shaking.
6. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair has been maintained, and the hair shape has not changed from before the treatment.

### <III: Imparting a semi-permanent straight shape to semi-permanent curly-shaped hair>

1. The tress, which had been evaluated in <II: Imparting a semi-permanent curly shape to semi-permanent straight-shaped hair> above, was combed to detangle it, and thereafter, a flat iron with an actual temperature of 180°C was slid through the tress at a rate of 5 cm/sec six times.
2. Tress was rinsed with running tap water at 30°C for 30 seconds, and a shampoo for evaluation was then lathered on the tress for 60 seconds. Thereafter, the tress was rinsed with running tap water at 30°C for 30 seconds, and was then dried with a towel.
3. The tress was dried, while being shaken, so that the natural shape of hair could appear (wherein dryer was not used), and it was then combed. The tress was hung, and was then visually observed from the side.

### (Evaluation criteria)

A: The curl has not remained, and the hair is completely deformed into straight hair.
B: The curl has become weaker than before the treatment with a flat iron, but the hair has not been completely deformed into straight hair.
C: The curly hair has been maintained, and the hair shape has not changed from before the treatment.

**[Table 2]**

| | | | | Example |
|---|---|---|---|---|
| | | | | 13 |
| Formulated first agent | Formulation [mass%] | (B) | (+)-Catechin | 9.9 |
| | | Others | Ethanol | 40 |
| | | | Sodium hydroxide | (*1) |
| | | (C) | Deionized water | Balance |
| | | Total | | 100 |
| | pH | | | 4 |
| Formulated second agent | Formulation [mass%] | (A) | Glyoxylic acid | 10 |
| | | Others | Sodium hydroxide | (*2) |
| | | (C) | Deionized water | Balance |
| | | Total | | 100 |
| | pH | | | 2 |
| Component molar ratio | | | (B)/(A) | 0.25 |
| Evaluation results | | | I. Imparting a semi-permanent straight shape | A |
| | | | II. Imparting a semi-permanent curly shape to semi-permanent straight-shaped hair | B |
| | | | III. Imparting a semi-permanent straight shape to semi-permanent curly-shaped hair | A |

| | | | | |
|---|---|---|---|---|
| *1: Amount for adjusting to pH 4.0 *2: Amount for adjusting to pH 2.0 | | | | |

### Example 14

The treatment agents shown in Table 3 were prepared, and the following three-step hair treatment process was then carried out using the agent. The shape-giving effect of each agent at each treatment was evaluated. The results are also shown in Table 3.

### <I: Imparting a semi-permanent curly shape>

1. A 25 cm-long tress, which consisted of 0.5 g of Caucasian straight hair, was wetted with tap water at 30°C for 30 seconds, and the wet tress was then wound around a plastic rod with a diameter of 14 mm, and fixed with a clip.
2. 1 g of the formulated first agent was applied to the tress wound around the rod, and the rod was then entirely covered with a plastic film for hermetical sealing. The tress was then heated for 1 hour in an oven, which was set at 90°C.
3. The tress was removed from the oven, and cooled to a room temperature.
4. The tress was removed from the plastic film, and the hair was then rinsed with running tap water at 30°C for 30 seconds. Thereafter, a shampoo for evaluation was lathered on the tress for 60 seconds. After that, the tress was rinsed with running tap water at 30°C for 30 seconds.
5. The wet tress was wound around a plastic rod with a diameter of 14 mm, and fixed with a clip.
6. 1.0 g of the formulated second agent was applied to the tress wound around the rod, and the rod was entirely covered with a plastic film for hermetical sealing, and heated for 1 hour in an oven, which was set at 90°C.
7. The tress was removed from the oven, and cooled to a room temperature.
8. The tress was removed from the rod, and was then rinsed with running tap water at 30°C for 30 seconds. Thereafter, a shampoo for evaluation was lathered on the tress for 60 seconds.
9. The tress was rinsed with running tap water at 30°C for 30 seconds, and immersed at an infinite bath ratio in tap water at 30°C for 60 seconds. Thereafter, the tress was gently pulled up out of the water while the root thereof being held, and water was then drained off by light shaking.
10. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

The formulation applied in Example 4 was identical to the formulation applied in Example 14. However, the touch feeling of the tress was much better in Example 14 than in Example 4.

### (Evaluation criteria)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair has been maintained, and the hair shape has not changed from before the treatment.

### <II: Imparting a semi-permanent straight shape to semi-permanent curly-shaped hair>

1. The tress, which had been evaluated in <I: Imparting a semi-permanent curly shape> above, was combed to detangle it, and a flat iron with an actual temperature of 180°C was then slid through the tress at a rate of 5 cm/sec six times.
2. The tress was rinsed with running tap water at 30°C for 30 seconds, and a shampoo for evaluation was then lathered on the tress for 60 seconds. Thereafter, the tress was rinsed with running tap water at 30°C for 30 seconds, and was then dried with a towel.
3. The tress was dried, while being shaken, so that the natural shape of hair could appear (wherein dryer was not used), and it was then combed. Thereafter, the tress was hung, and was then visually observed from the side.

### (Evaluation criteria)

A: The curl has not remained, and the hair has been completely deformed into straight hair.
B: The curl has become weaker than before the treatment with a flat iron, but the hair has not been completely deformed into straight hair.
C: The curly hair has remained, and the hair shape has not changed from before the treatment.

### <III: Imparting a semi-permanent curly shape to semi-permanent straight-shaped hair>

1. The tress, which had been evaluated in <II: Imparting a semi-permanent straight shape to semi-permanent curly-shaped hair> above, was wetted with tap water at 30°C for 30 seconds, and the wet tress was then wound around a plastic rod with a diameter of 14 mm, and fixed with a clip.
2. The rod was entirely covered with a plastic film for hermetical sealing. Thereafter, the tress was heated for 1 hour in an oven, which was set at 40°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and immersed at an infinite bath ratio in tap water at 30°C for 60 seconds. Thereafter, the tress was gently pulled up out of the water while the root thereof being held, and water was then drained off by light shaking.
6. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair has been maintained, and the hair shape has not changed from before the treatment.

**[Table 3]**

| | | | | Example |
|---|---|---|---|---|
| | | | | 14 |
| Formulated first agent | Formulation [mass%] | (B) | (+)-Catechin | 9.9 |
| | | Others | Ethanol | 40 |
| | | | Sodium hydroxide | (*1) |
| | | (C) | Deionized water | Balance |
| | | Total | | 100 |
| | pH | | | 4 |
| Formulated second agent | Formulation [mass%] | (A) | Glyoxylic acid | 10 |
| | | Others | Sodium hydroxide | (*2) |
| | | (C) | Deionized water | Balance |
| | | Total | | 100 |
| | pH | | | 2 |
| Component molar ratio | | | (B)/(A) | 0.25 |
| Evaluation results | | | I. Imparting a semi-permanent curly shape | A |
| | | | II. Imparting a semi-permanent straight shape to semi-permanent curly-shaped hair | A |
| | | | III. Imparting a semi-permanent curly shape to semi-permanent straight-shaped hair | A |

| | | | | |
|---|---|---|---|---|
| *1: Amount for adjusting to pH 4.0 *2: Amount for adjusting to pH 2.0 | | | | |

### Comparative Example 5

A test was carried out in accordance with Example 1 described in Patent Document 2 (International Publication No. WO 2009/035970). Specifically, a composition shown in Table 4 below was prepared, and the following hair treatment was carried out using the composition. Thereafter, the effect of giving a shape to hair was evaluated.

### <I: Imparting a semi-permanent straight shape>

1. A tress consisting of slightly spread, straight hair from Caucasian race (untreated hair) (weight: 0.5 g / length: 2.5 cm) was wetted with tap water at 30°C for 30 seconds, and the wet tress was then placed on a plastic film. The tress was linearly arranged thereon.
2. 1.0 g of the treatment agent was applied to the tress, and it was evenly spread on the hair. Thereafter, the entire tress was covered with a plastic film for hermetically sealing, and it was then left at a room temperature for 30 minutes.
3. The tress was removed from the plastic film, and water was lightly removed with a towel. Thereafter, the tress was dried with hot air from a dryer, until it was completely dried.
4. The tress was combed to detangle it, and a flat iron with an actual temperature of 230°C was slid through the tress at a rate of 7.5 cm/sec ten times, so that the style of straight hair was completely formed from the roots to the tips of the tress.
5. The tress was rinsed with running tap water at 30°C for 30 seconds. Thereafter, a shampoo for evaluation was lathered on the tress for 60 seconds. After that, the tress was rinsed with running tap water at 30°C for 30 seconds, and was then dried with a towel.
6. The tress was dried, while being shaken, so that the shape of hair could directly appear, and it was then combed. Thereafter, the tress was hung, and was then visually observed from the side.

### (Evaluation criteria)

A: The spreading of the tress disappears, and completely straight hair is maintained from the roots to the tips of the tress.
B: Although the tress is straight hair, in which the spreading of the tress is suppressed, it is slightly spread.
C: The spreading of the tress is equivalent to that of untreated hair.

### <II: Imparting a semi-permanent curly shape to semi-permanent straight-shaped hair>

1. The tress, which had been evaluated in <I: Imparting a semi-permanent straight shape> above, was wetted with tap water at 30°C for 30 seconds, and the wet tress was then wound around a plastic rod with a diameter of 14 mm, and fixed with a clip.
2. The rod was entirely covered with a plastic film for hermetical sealing. Thereafter, the tress was heated in an oven, which was set at 40°C, for 1 hour.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and immersed at an infinite bath ratio in tap water at 30°C for 60 seconds. Thereafter, the tress was gently pulled up out of the water while the root thereof being held, and water was then drained off by light shaking.
6. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair has been maintained, and the hair shape has not changed from before the treatment.

As shown in Table 4, the hair treatment agent of the comparative example could not provide a curly shape to hair, after it had given a semi-permanent straight shape to the hair. Thus, the hair treatment agent of the comparative example did not have the effect of the present invention, by which hair can be deformed into any given shape by heating the hair.

**[Table 4]**

| | | | Comparative Example |
|---|---|---|---|
| | | | 5 |
| Formulation [mass%] | (A') | Glyoxal | 5 |
| | (B) | Epigallocatechin gallate | 3 |
| | Others | Sodium hydroxide | (*1) |
| | (C) | Deionized water | Balance |
| | Total | | 100 |
| pH | | | 3.0 |
| Component molar ratio | | (B)/(A') | 0.076 |
| Evaluation results | | I. Imparting a semi-permanent straight shape | B |
| | | II. Imparting a semi-permanent curly shape to semi-permanent straight-shaped hair | E |
| | | III. Imparting a semi-permanent straight shape to semi-Dermanent curly-shaped hair | (*2) |

| | | | |
|---|---|---|---|
| *1: Amount for adjusting to pH 3.0 *2: Since curly shape could not be given to hair in Evaluation II, evaluation was not conducted. | | | |

## Claims

1. An agent for hair deforming treatment comprising the following components (A), (B) and (C):
(A) glyoxylic acid, or a hydrate or a salt thereof,
(B) one or two or more member selected from the compounds represented by the following formula (1): 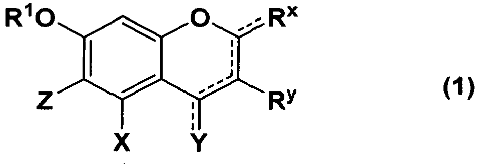 wherein
R¹ represents a hydrogen atom or a methyl group,
X represents a hydrogen atom, a hydroxy group, or a methoxy group,
Y represents a hydrogen atom, an oxygen atom, a hydroxy group, or a methoxy group,
Z represents a hydrogen atom, or a straight-chain or branched-chain alkyl group or alkenyl group containing from 1 to 5 carbon atoms,
R^{x} represents a hydrogen atom, an oxygen atom, a hydroxy group, a methoxy group, or an aromatic hydrocarbon group, which is optionally substituted with up to three hydroxy groups or methoxy groups and which optionally forms a condensed ring with 1,3-dioxolane,
R^{y} represents a hydrogen atom, a hydroxy group, a methoxy group, or an aromatic hydrocarbon group, which is optionally substituted with up to three hydroxy groups or methoxy groups and which optionally forms a condensed ring with 1,3-dioxolane, or an arylcarbonyloxy group or aralkylcarbonyloxy group, which is optionally substituted with up to three hydroxy groups or methoxy groups, and
the dashed line represents that the portion is optionally a double bond,
provided that the dashed line and the solid line, which are adjacent to R^{x} or Y, each represent a double bond only when R^{x} or Y represents an oxygen atom, and otherwise these lines each represent a single bond, and
provided that Z is a straight-chain or branched-chain alkyl group or alkenyl group containing from 1 to 5 carbon atoms only when R^{x} or R^{y} is an o,p-dihydroxy aromatic hydrocarbon group, and otherwise Z is a hydrogen atom, and
(C) water,
wherein the molar ratio of the content of the component (B) to the content of the component (A), (B)/(A), is 0.01 or more and 5 or less; and
wherein the content of the component (A) is 2.5 mass% or more and 30 mass% or less.

2. The agent for hair deforming treatment according to claim 1, wherein the molar ratio of the content of the component (B) to the content of the component (A), (B)/(A), is 0.1 or more and 2 or less.

3. The agent for hair deforming treatment according to claim 1 or 2, wherein the component (B) is a compound represented by the following formula (1-1): 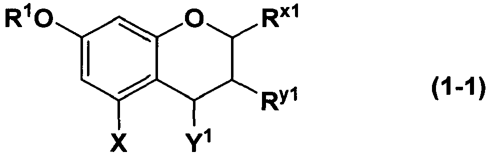 wherein
R¹ and X are as defined above,
Y¹ represents a hydrogen atom, a hydroxy group, or a methoxy group,
R^{x1} represents an aromatic hydrocarbon group, which is optionally substituted with up to three hydroxy groups or methoxy groups and which optionally forms a condensed ring with 1,3-dioxolane, and
R^{y1} represents a hydrogen atom, a hydroxy group, a methoxy group, or an aromatic hydrocarbon group, which is optionally substituted with up to three hydroxy groups or methoxy groups and which optionally forms a condensed ring with 1,3-dioxolane, or an arylcarbonyloxy group or aralkylcarbonyloxy group, which is optionally substituted with up to three hydroxy groups or methoxy groups.

4. The agent for hair deforming treatment according to any one of claims 1 to 3, wherein the component (B) is one or two or more member selected from the group consisting of catechin, epicatechin, epigallocatechin, meciadanol, afzelechin, epiafzelechin, catechin gallate, epicatechin gallate, epigallocatechin gallate, phylloflavan, and fisetinidol.

5. The agent for hair deforming treatment according to any one of claims 1 to 4, wherein the content of the component (B) is 0.2 mass% or more and 30 mass% or less.

6. The agent for hair deforming treatment according to any one of claims 1 to 5, wherein the pH thereof is 4 or less.

7. The agent for hair deforming treatment according to any one of claims 1 to 6, which is a multi-part agent comprising a first agent comprising the component (B) and a second agent comprising the component (A), wherein the pH of the second agent is 4 or less.

8. The agent for hair deforming treatment according to claim 7, wherein the pH of the first agent is 2.5 or more and 6 or less, and the pH of the second agent is 1 or more and 4 or less.

9. The agent for hair deforming treatment according to any one of claims 1 to 8, wherein the total amount of components for reducing hair proteins is less than 0.1 mass%.

10. A method of hair treatment for semi-permanently or permanently deforming the shape of hair, which comprises the following steps (i) and (ii):
(i) applying the agent for hair deforming treatment according to any one of claims 1 to 9 to hair, and then allowing the agent to penetrate into the hair, and
(ii) heating and shaping the hair into which the agent for hair deforming treatment has penetrated.

11. The method of hair treatment according to claim 10, wherein the step (i) is a step of applying one of the first agent and the second agent in the agent for hair deforming treatment according to claim 8 to hair, and then applying the other part onto the portion applied.

12. The method of hair treatment according to claim 11, which does not comprise a step of rinsing the first agent after application of the first agent and before application of the second agent, wherein the molecular weight of the component (B) comprised in the first agent is 100 or more and 180 or less.

13. The method of hair treatment according to claim 11, which comprises a step of rinsing the first agent after application of the first agent and before application of the second agent, wherein the molecular weight of the component (B) comprised in the first agent is 140 to 1000.

14. The method of hair treatment according to any one of claims 10 to 13, which comprises a step of wetting hair before the step (i).

15. The method of hair treatment according to any one of claims 10 to 14, wherein the heating temperature in the step (ii) is 50°C or higher and 250°C or lower.

16. The method of hair treatment according to any one of claims 10 to 15, wherein the step (ii) is carried out under an environment in which evaporation of water is suppressed.

17. The method of hair treatment according to any one of claims 10 to 16, which does not comprise a step of applying a hair treatment agent comprising a reducing agent or a hair treatment agent having pH 12 to 14 to the hair.

18. The method of hair treatment according to any one of claims 10 to 17, which comprises a step of heating the hair again and deforming it, after the step (ii).

## Patentansprüche

1. Behandlungsmittel zur Haarverformung, umfassend die folgenden Komponenten (A), (B) und (C):
(A) Glyoxylsäure, oder ein Hydrat oder ein Salz davon,
(B) ein oder zwei oder mehr Vertreter ausgewählt aus den Verbindungen, die durch die folgende Formel (1) dargestellt werden: 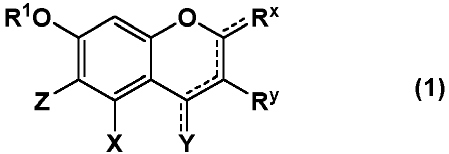 wobei
R¹ ein Wasserstoffatom oder eine Methylgruppe darstellt,
X ein Wasserstoffatom, eine Hydroxygruppe oder eine Methoxygruppe darstellt,
Y ein Wasserstoffatom, ein Sauerstoffatom, eine Hydroxygruppe oder eine Methoxygruppe darstellt,
Z ein Wasserstoffatom oder eine geradkettige oder eine verzweigtkettige Alkylgruppe oder Alkenylgruppe mit 1 bis 5 Kohlenstoffatomen darstellt,
R^{x} ein Wasserstoffatom, ein Sauerstoffatom, eine Hydroxygruppe, eine Methoxygruppe oder eine aromatische Kohlenwasserstoffgruppe, die optional mit bis zu drei Hydroxygruppen oder Methoxygruppen substituiert ist und die optional mit 1,3-Dioxolan einen kondensierten Ring bildet, darstellt,
R^{y} ein Wasserstoffatom, eine Hydroxygruppe, eine Methoxygruppe oder eine aromatische Kohlenwasserstoffgruppe, die optional mit bis zu drei Hydroxygruppen oder Methoxygruppen substituiert ist und die optional mit 1,3-Dioxolan einen kondensierten Ring bildet, oder eine Arylcarbonyloxygruppe oder Aralkylcarbonyloxygruppe, die optional mit bis zu drei Hydroxygruppen oder Methoxygruppen substituiert ist, darstellt, und
die gestrichelte Linie darstellt, dass der Abschnitt gegebenenfalls eine Doppelbindung ist,
vorausgesetzt, dass die gestrichelte Linie und die durgezogene Linie, die an R^{x} oder Y angrenzen, jeweils nur dann eine Doppelbindung darstellen, wenn R^{x} oder Y ein Sauerstoffatom darstellen, und diese Linien anderenfalls eine Einfachbindung darstellen, und
vorausgesetzt, dass Z nur eine geradkettige oder verzweigtkettige Alkylgruppe oder Alkenylgruppe mit 1 bis 5 Kohlenstoffatome ist, wenn R^{x} oder R^{y} eine aromatische o,p-Dihydroxykohlenwasserstoffgruppe ist, und anderenfalls Z ein Wasserstoffatom ist, und
(C) Wasser,
wobei das molare Verhältnis des Gehalts der Komponente (B) zu dem Gehalt der Komponente (A), (B)/(A), 0,01 oder mehr und 5 oder weniger beträgt; und
wobei der Gehalt der Komponente (A) 2.5 Massen% oder mehr und 30 Massen% oder weniger beträgt.

2. Behandlungsmittel zur Haarverformung gemäß Anspruch 1, wobei das molare Verhältnis des Gehalts der Komponente (B) zu dem Gehalt der Komponente (A), (B)/(A), 0,1 oder mehr und 2 oder weniger beträgt.

3. Behandlungsmittel zur Haarverformung gemäß Anspruch 1 oder 2, wobei die Komponente (B) eine Verbindung ist, die durch die folgende Formel (1-1) dargestellt wird: 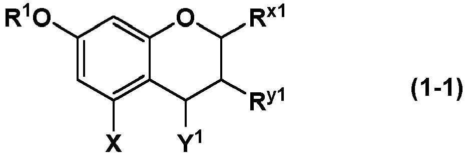 wobei
R¹ und X wie oben definiert sind,
Y¹ ein Wasserstoffatom, eine Hydroxygruppe oder eine Methoxygruppe darstellt,
R^{x1} eine aromatische Kohlenwasserstoffgruppe darstellt, die optional mit bis zu drei Hydroxygruppen oder Methoxygruppen substituiert ist und die optional mit 1,3-Dioxolan einen kondensierten Ring bildet,
R^{y1} ein Wasserstoffatom, eine Hydroxygruppe, eine Methoxygruppe, oder eine aromatische Kohlenwasserstoffgruppe, die optional mit bis zu drei Hydroxygruppen oder Methoxygruppen substituiert ist und die optional mit 1,3-Dioxolan einen kondensierten Ring bildet, oder eine Arylcarbonyloxygruppe oder Aralkylcarbonyloxygruppe, die optional mit bis zu drei Hydroxygruppen oder Methoxygruppen substituiert ist, darstellt.

4. Behandlungsmittel zur Haarverformung gemäß einem der Ansprüche 1 bis 3, wobei die Komponente (B) ein oder zwei oder mehrere Vertreter, ausgewählt aus der Gruppe bestehend aus Catechin, Epicatechin, Epigallocatechin, Meciadanol, Afzelechin, Epiafzelechin, Catechingallat, Epicatechingallat, Epigallocatechingallat, Phylloflavan und Fisetinidol, ist.

5. Behandlungsmittel zur Haarverformung gemäß einem der Ansprüche 1 bis 4, wobei der Gehalt der Komponente (B) 0,2 Massen% oder mehr und 30 Massen% oder weniger beträgt.

6. Behandlungsmittel zur Haarverformung gemäß einem der Ansprüche 1 bis 5, dessen pH 4 oder weniger beträgt.

7. Behandlungsmittel zur Haarverformung gemäß einem der Ansprüche 1 bis 6, das ein Mehrkomponentenmittel ist umfassend ein erstes Mittel, das die Komponente (B) umfasst, und ein zweites Mittel, das die Komponente (A) umfasst, wobei der pH des zweiten Mittels 4 oder weniger beträgt.

8. Behandlungsmittel zur Haarverformung gemäß Anspruch 7, wobei der pH des ersten Mittels 2,5 oder mehr und 6 oder weniger beträgt, und der pH des zweiten Mittels 1 oder mehr und 4 oder weniger beträgt.

9. Behandlungsmittel zur Haarverformung gemäß einem der Ansprüche 1 bis 8, wobei die Gesamtmenge der Komponenten zur Reduktion von Haarproteinen weniger als 0,1 Massen% beträgt.

10. Verfahren zur Behandlung von Haaren zur semipermanenten oder permanenten Verformung der Form der Haare, welches die folgenden Schritte (i) und (ii) umfasst:
(i) Aufbringen des Behandlungsmittels zur Haarverformung gemäß einem der Ansprüche 1 bis 9 auf Haar, und anschließendes Eindringenlassen des Mittels in die Haare, und
(ii) Erwärmen und Formen der Haare, in welche das Behandlungsmittel zur Haarverformung eingedrungen ist.

11. Verfahren zur Behandlung von Haaren gemäß Anspruch 10, wobei der Schritt (i) ein Schritt ist, indem eines des ersten Mittels und des zweiten Mittels des Behandlungsmittel zur Haarverformung gemäß Anspruch 8 auf die Haare aufgebracht wird und anschließend der andere Teil auf den behandelten Bereich aufgebracht wird.

12. Verfahren zur Behandlung von Haaren gemäß Anspruch 11, das keinen Schritt des Ausspülens des ersten Mittels nach dem Aufbringen des ersten Mittels und vor dem Aufbringen des zweiten Mittels umfasst, wobei das Molekulargewicht der Komponente (B), die im ersten Mittel enthalten ist, 100 oder mehr und 180 oder weniger beträgt.

13. Verfahren zur Behandlung von Haaren gemäß Anspruch 11, das einen Schritt des Ausspülens des ersten Mittels nach dem Aufbringen des ersten Mittels und vor dem Aufbringen des zweiten Mittels umfasst, wobei das Molekulargewicht der Komponente (B), die im ersten Mittel enthalten ist, 140 bis 1000 beträgt.

14. Verfahren zur Behandlung von Haaren gemäß einem der Ansprüche 10 bis 13, das einen Schritt des Befeuchtens der Haare vor dem Schritt (i) umfasst.

15. Verfahren zur Behandlung von Haaren gemäß einem der Ansprüche 10 bis 14, wobei die Erwärmungstemperatur im Schritt (ii) 50 °C oder mehr und 250 °C oder weniger beträgt.

16. Verfahren zur Behandlung von Haaren gemäß einem der Ansprüche 10 bis 15, wobei der Schritt (ii) unter einer Atmosphäre durchgeführt wird, in der die Verdunstung von Wasser unterdrückt wird.

17. Verfahren zur Behandlung von Haaren gemäß einem der Ansprüche 10 bis 16, das keinen Schritt des Aufbringens eines Haarbehandlungsmittels, das ein Reduktionsmittel umfasst, oder eines Haarbehandlungsmittels, das einen pH von 12 bis 14 aufweist, auf die Haare umfasst.

18. Verfahren zur Behandlung von Haaren gemäß einem der Ansprüche 10 bis 17, das einen Schritt des erneuten Erwärmens und Verformens der Haare nach dem Schritt (ii) umfasst.

## Revendications

1. Agent pour traitement de déformation capillaire comprenant les composants (A), (B) et (C) suivants :
(A) acide glyoxylique, ou un hydrate ou un sel de celui-ci,
(B) un ou deux ou plus de deux membres choisis parmi les composés représentés par la formule (1) suivante : 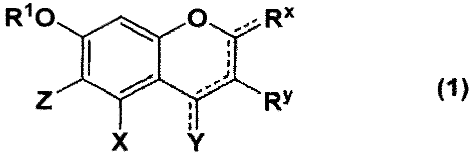 où
R¹ représente un atome d'hydrogène ou un groupe méthyle,
X représente un atome d'hydrogène, un groupe hydroxy, ou un groupe méthoxy,
Y représente un atome d'hydrogène, un atome d'oxygène, un groupe hydroxy, ou un groupe méthoxy,
Z représente un atome d'hydrogène, ou un groupe alkyle ou groupe alcényle à chaîne rectiligne ou à chaîne ramifiée contenant de 1 à 5 atomes de carbone,
R^{x} représente un atome d'hydrogène, un atome d'oxygène, un groupe hydroxy, un groupe méthoxy, ou un groupe hydrocarboné aromatique, qui est facultativement substitué par jusqu'à trois groupes hydroxy ou groupes méthoxy et qui forme facultativement un cycle condensé avec du 1,3-dioxolane,
R^{y} représente un atome d'hydrogène, un groupe hydroxy, un groupe méthoxy, ou un groupe hydrocarboné aromatique, qui est facultativement substitué par jusqu'à trois groupes hydroxy ou groupes méthoxy et qui forme facultativement un cycle condensé avec du 1,3-dioxolane, ou un groupe arylcarbonyloxy ou groupe aralkylcarbonyloxy, qui est facultativement substitué par jusqu'à trois groupes hydroxy ou groupes méthoxy, et
la ligne en pointillés représente que la partie est facultativement une liaison double,
à condition que la ligne en pointillés et la ligne continue, qui sont adjacentes à R^{x} ou Y, représentent chacune une liaison double uniquement lorsque R^{x} ou Y représente un atome d'oxygène, et sinon ces lignes représentent chacune une liaison simple, et
à condition que Z soit un groupe alkyle ou groupe alcényle à chaîne rectiligne ou à chaîne ramifiée contenant de 1 à 5 atomes de carbone uniquement lorsque R^{x} ou R^{y} est un groupe hydrocarboné aromatique o,p-dihydroxy, et sinon Z est un atome d'hydrogène, et
(C) de l'eau,
dans lequel le rapport molaire entre la teneur en composant (B) et la teneur en composant (A), (B)/(A), est de 0,01 ou plus et de 5 ou moins ; et
dans lequel la teneur en composant (A) est de 2,5 % en masse ou plus et de 30 % en masse ou moins.

2. Agent pour traitement de déformation capillaire selon la revendication 1, dans lequel le rapport molaire entre la teneur en composant (B) et la teneur en composant (A), (B)/(A), est de 0,1 ou plus et de 2 ou moins.

3. Agent pour traitement de déformation capillaire selon la revendication 1 ou 2, dans lequel le composant (B) est un composé représenté par la formule (1-1) suivante : où 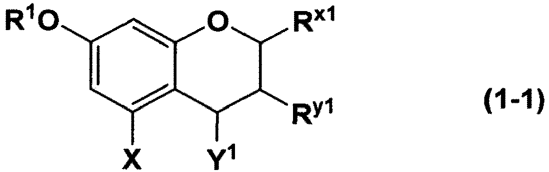
R¹ et X sont tels que définis ci-dessus,
Y¹ représente un atome d'hydrogène, un groupe hydroxy, ou un groupe méthoxy,
R^{x1} représente un groupe hydrocarboné aromatique, qui est facultativement substitué par jusqu'à trois groupes hydroxy ou groupes méthoxy et qui forme facultativement un cycle condensé avec du 1,3-dioxolane, et
R^{Y1} représente un atome d'hydrogène, un groupe hydroxy, un groupe méthoxy, ou un groupe hydrocarboné aromatique, qui est facultativement substitué par jusqu'à trois groupes hydroxy ou groupes méthoxy et qui forme facultativement un cycle condensé avec du 1,3-dioxolane, ou un groupe arylcarbonyloxy ou groupe aralkylcarbonyloxy, qui est facultativement substitué par jusqu'à trois groupes hydroxy ou groupes méthoxy.

4. Agent pour traitement de déformation capillaire selon l'une quelconque des revendications 1 à 3, dans lequel le composant (B) est un ou deux ou plus de deux membres choisis parmi le groupe constitué de catéchine, épicatéchine, épigallocatéchine, méciadanol, afzéléchine, épiafzéléchine, gallate de catéchine, gallate d'épicatéchine, gallate d'épigallocatéchine, phylloflavan, et fisétinidol.

5. Agent pour traitement de déformation capillaire selon l'une quelconque des revendications 1 à 4, dans lequel la teneur en composant (B) est de 0,2 % en masse ou plus et de 30 % en masse ou moins.

6. Agent pour traitement de déformation capillaire selon l'une quelconque des revendications 1 à 5, dans lequel son pH est de 4 ou moins.

7. Agent pour traitement de déformation capillaire selon l'une quelconque des revendications 1 à 6, qui est un agent en plusieurs parties comprenant un premier agent comprenant le composant (B) et un second agent comprenant le composant (A), dans lequel le pH du second agent est de 4 ou moins.

8. Agent pour traitement de déformation capillaire selon la revendication 7, dans lequel le pH du premier agent est de 2,5 ou plus et de 6 ou moins, et le pH du second agent est de 1 ou plus et de 4 ou moins.

9. Agent pour traitement de déformation capillaire selon l'une quelconque des revendications 1 à 8, dans lequel la quantité totale de composants pour réduire des protéines capillaires est inférieure à 0,1 % en masse.

10. Procédé de traitement capillaire pour déformer de façon semi-permanente ou permanente la forme de cheveux, qui comprend les étapes (i) et (ii) suivantes consistant à :
(i) appliquer l'agent pour traitement de déformation capillaire selon l'une quelconque des revendications 1 à 9 sur des cheveux, et laisser ensuite l'agent pénétrer dans les cheveux, et
(ii) chauffer et mettre en forme les cheveux dans lesquels l'agent pour traitement de déformation capillaire a pénétré.

11. Procédé de traitement capillaire selon la revendication 10, dans lequel l'étape (i) est une étape consistant à appliquer l'un des premier et second agents dans l'agent pour traitement de déformation capillaire selon la revendication 8 sur des cheveux, et à appliquer ensuite l'autre partie sur la portion appliquée.

12. Procédé de traitement capillaire selon la revendication 11, qui ne comprend pas une étape de rinçage du premier agent après application du premier agent et avant application du second agent, dans lequel le poids moléculaire du composant (B) compris dans le premier agent est de 100 ou plus et de 180 ou moins.

13. Procédé de traitement capillaire selon la revendication 11, qui comprend une étape de rinçage du premier agent après application du premier agent et avant application du second agent, dans lequel le poids moléculaire du composant (B) compris dans le premier agent est de 140 à 1000.

14. Procédé de traitement capillaire selon l'une quelconque des revendications 10 à 13, qui comprend une étape de mouillage des cheveux avant l'étape (i).

15. Procédé de traitement capillaire selon l'une quelconque des revendications 10 à 14, dans lequel la température de chauffage à l'étape (ii) est de 50 °C ou plus et de 250 °C ou moins.

16. Procédé de traitement capillaire selon l'une quelconque des revendications 10 à 15, dans lequel l'étape (ii) est réalisée dans un environnement dans lequel une évaporation d'eau est atténuée.

17. Procédé de traitement capillaire selon l'une quelconque des revendications 10 à 16, qui ne comprend pas une étape d'application d'un agent de traitement capillaire comprenant un agent réducteur ou un agent de traitement capillaire ayant un pH de 12 à 14 sur les cheveux.

18. Procédé de traitement capillaire selon l'une quelconque des revendications 10 à 17, qui comprend une étape consistant à chauffer à nouveau les cheveux et à les déformer, après l'étape (ii).
